# EUROPEAN PATENT SPECIFICATION

(11) **EP 1 763 365 B1**
(45) Date of publication and mention of the grant of the patent: **10.08.2016**
(21) Application number: 05750202.3
(22) Date of filing: 09.06.2005
(51) Int. Cl.: A61K 39/395, A61P 35/00

(54) **ANTIBODIES FOR SELECTIVE APOPTOSIS OF CELLS**
ANTIKÖRPER ZUR SELEKTIVEN APOPTOSE VON ZELLEN
ANTICORPS POUR APOPTOSE SELECTIVE DE CELLULES

(30) Priority: 09.06.2004 US 577920 P
(43) Date of publication of application: 21.03.2007
(73) Proprietor: TECHNION RESEARCH AND DEVELOPMENT FOUNDATION, LTD., Haifa 32000 (IL)
(72) Inventor: REITER, Yoram, 34790 Haifa (IL); KLECHEVSKY, Eynav, 34817 Haifa (IL); DENKBERG, Galit, 36001 Nofit (IL)
(74) Representative: Dennemeyer & Associates S.A.
(86) International application number: PCT/IL2005/000616
(87) International publication number: WO 2005/120166

(56) References cited:
- WO-A-03/068201
- DENKBERG GALIT ET AL: "Selective targeting of melanoma and APCs using a recombinant antibody with TCR-like specificity directed toward a melanoma differentiation antigen." JOURNAL OF IMMUNOLOGY (BALTIMORE, MD. : 1950) 1 SEP 2003, vol. 171, no. 5, 1 September 2003 (2003-09-01), pages 2197-2207, XP002383418 ISSN: 0022-1767
- AZRIEL-ROSENFELD R ET AL: "A Human Synthetic Combinatorial Library of Arrayable Single-chain Antibodies based on Shuffling in Vivo Formed CDRs into General Framework Regions" JOURNAL OF MOLECULAR BIOLOGY, LONDON, GB, vol. 335, no. 1, 2 January 2004 (2004-01-02), pages 177-192, XP004476455 ISSN: 0022-2836 cited in the application
- CHAMES PATRICK ET AL: "TCR-like human antibodies expressed on human CTLs mediate antibody affinity-dependent cytolytic activity." JOURNAL OF IMMUNOLOGY (BALTIMORE, MD. : 1950) 15 JUL 2002, vol. 169, no. 2, 15 July 2002 (2002-07-15), pages 1110-1118, XP002383419 ISSN: 0022-1767
- SKOV S ET AL: "Ligation of major histocompatability complex (MHC) class I molecules on human T cells induces cell death through PI-3 kinase-induced c-Jun NH2-terminal kinase activity: a novel apoptotic pathway distinct from Fas-induced apoptosis." THE JOURNAL OF CELL BIOLOGY. 15 DEC 1997, vol. 139, no. 6, 15 December 1997 (1997-12-15), pages 1523-1531, XP002383420 ISSN: 0021-9525 cited in the application
- NOY ROY ET AL: "T-cell receptor-like antibodies: novel reagents for clinical cancer immunology and immunotherapy." EXPERT REVIEW OF ANTICANCER THERAPY. JUN 2005, vol. 5, no. 3, June 2005 (2005-06), pages 523-536, XP009067037 ISSN: 1473-7140

## Description

The present disclosure is of recombinant isolated antibodies which specifically recognize MHC-peptide complexes and are capable of inducing apoptosis in a peptide-specific MHC-restricted manner, and more particularly, of methods of treating and diagnosing cancer or pathogen infections using such recombinant and isolated, TCR-like antibodies.

Ligation of several surface receptors are known to induce apoptosis. In activated B cells, Fas ligation induces apoptosis by activating a number of specific caspases. In addition, cross-linking of B cell receptors initiates a distinct apoptotic pathway at certain stages of B cell development.

The Major Histocompatibility Complex class I (MHC-I) plays a central role in human immune system, by acting as a framework for peptide presentation to CD81 T-cells (Boon T. van der B.P., 1996). MHC-I molecules were recently acknowledged as important signal transducing molecules involved in regulation and fine-tuning of immune responses. For example, while cross-linking of the α3 domain of MHC-I induced apoptosis in resting B cells, the anti-MHC-I antibody (Ab), BAL-1, induced apoptosis in activated B cells. While the apoptotic pathway induced by MHC-I ligation of T cells is distinctly different from that of Fas, the apoptotic induced by MHC-I cross-linking in B cells is not fully understood. One of the events which follows MHC-I cross-linking is mobilization of intracellular calcium. Thus, cross linking of MHC-I molecules using an anti-β2 microglobulin antibody (Ab) resulted in protein tyrosine phosphorylation of the p53/56lyn and p72syk protein tyrosine kinases, leading to an increase in intracellular Calcium (Nagy ZA, et al., 2002; Nagy ZA, et al., 2003; Longo, D.L., 2002. Vidovic D, Toral JI., 1998; Pedersen AE, et al., 1999; Ruhwald M, et al., 1999; Mori M, et al., 1999; Genestier L, et al., 1997; Genestier L, et al., 1997; Skov S, et al., 1997).

A significant progress in understanding the mechanisms leading to cellular immune response against tumor cells was achieved by the characterization of MHC-displayed tumor-associated antigens and the advent of appropriate methodology developed to monitor immune recognition [Altman et. al., 1996; Boon, 1996; Lee et. al., 1999; Rosenberg, 2001; Renkvist et. al., 2001]. Cytotoxic T lymphocytes recognize antigens as short peptides bound to MHC-I molecules [Klein and Sato, 2000]. Most MHC-I - presented peptides are derived from degradation of intracellular proteins by the proteasome [Niedermann, 2002]. Cleaved peptides are transported into the lumen of the endoplasmic reticulum (ER) by a transporter associated with a processing molecule (TAP), possibly protected by chaperone heat-shock proteins from complete degradation before entering the ER. The trimmed peptides are bound to the groove of the assembled MHC-I molecule in the ER, and the complex is then transported to the cell surface [Yewdell, 2001; Yewdell and Bennink., 2001]. The selection of peptides and their presentation on the cell surface depends on various factors such as the presence of endogenous or exogenous proteins in intracellular compartments, the appropriate degradation of such proteins in intracellular compartments, the ability of the degraded peptides to bind to the groove of the particular HLA molecule, and the successful transport of such molecules to the cell surface.

Several studies demonstrated that the inability of the patient's immune system to elicit an effective immune response against the tumor is often due to poor antigen presentation [Restifo et. al., 1993; Seliger et. al., 2000].

To study MHC-peptide presentation on the surface of macrophages, dendritic cells, tumor cells or virus-infected cells, recombinant soluble T-cell receptors (TCRs) were produced in *E. coli.* However, such TCRs exhibited instability and inherently low affinity for ligands [Wulfing and Pluckthun., 1994]. Thus, it was acknowledged that antibodies that would recognize tumor-associated MHC-peptide complexes with the same specificity as the TCR would be valuable reagents for studying antigen presentation by tumor cells, for visualizing MHC-peptide complexes on cells, and eventually for monitoring the expression of specific complexes during immunotherapy. In addition, they may serve as good candidates for targeting reagents in constructing recombinant immunotoxins, fusions with cytokine molecules, or for bi-specific antibody therapy.

Antibodies which specifically recognize class I peptide-MHC complexes were prepared in murine systems [Murphy et. al., 1989; Aharoni et. al., 1991; Andersen et. al., 1996; Reiter et. al., 1997; Porgador et. al., 1997; Day et. al., 1997; Dadaglio et. al., 1997; Zhong et. al., 1997a; Zhong et. al., 1997b; Krogsgaard et. al., 2000; Polakova et. al., 2000]. However, antibodies with such exquisite T-cell receptor-like specificity have proven difficult to produce using conventional hybridoma techniques or immunization strategies even in combination with *in vitro* selection from phage display libraries. The direct selection of such antibodies from very large non-immune phage antibody libraries was only recently demonstrated [Chames et. al., 2000; Proc. Natl. Acad. Sci USA. 97(14): 7969-74]. Using this route, a phage display-derived Fab was isolated (i.e., phage associated) that recognizes the melanoma antigen MAGE-A1 in complex with the human HLA-A1 MHC molecule. However, such an antibody (G8) exhibited a low affinity towards the MHC-complex in the range of 250 nM (Chames P., et al., 2002, J. Immunol. 169:1110-8). To overcome the low affinity limitation, Chames P., et al. (2002; J. Immunol. 169:1110-8) engrafted T cellsexpressing *in vitro* engineered G8 antibody and prepared cell-specific antibodies. However, such antibodies are impractical for treating cancer or pathogen infection since they require gene therapy manipulations on a patient-specific basis.

The present inventors have previously isolated TCR like antibodies from a murine scFv phage display library constructed from HLA-A2 transgenic mice which were immunized with the stable single-chain HLA-A2-β2 molecule complexed with a peptide derived from the melanoma differentiation Ag gp100 (Denkberg G., et al., The Journal of Immunology, 2003; 171(5):2197-207). In contrast to prior art attempts, this soluble, secreted, scFv recombinant antibody (G1) exhibited a high affinity (within the nanomolar range, e.g., 5 nM) to the HLA-A2-G9-209 complex, in a peptide specific, MHC-restricted manner, similar to the specificity of TCRs (Denkberg G., 2003, Supra).
WO 03068201 relates to an isolated molecule which comprises an antibody specifically bindable with a binding affinity below 20 nanomolar, preferably below 10 nanomolar, to a human major histocompatibility complex (MHC) class I being complexed with a HLA-restricted antigen and optionally further comprises an identifiable or therapeutic moiety conjugated to the antibody.

While reducing the present invention to practice, the present inventors have uncovered that isolated recombinant antibodies specifically recognizing MHC-peptide complexes can be used as potent apoptosis inducing agents and thus treat cancer or pathogen infection.

### SUMMARY OF THE INVENTION

The present invention relates to an *in vitro* method of inducing apoptosis in cancer cells, the method comprising contacting the cancer cells with a recombinant, isolated toxin-free single chain antibody capable of specifically binding an MHC class I-peptide complex specifically expressed on the cancer cells, thereby inducing apoptosis in the cancer cells.

The present invention also relates to the use of a recombinant, isolated toxin-free antibody capable of specifically binding an MHC class I-peptide complex, said recombinant, isolated toxin-free antibody being capable of inducing apoptosis of cancer cells specifically expressing said MHC-class I-peptide complex, wherein said recombinant, isolated toxin-free antibody is selected from the group consisting of a Fab fragment and a single chain antibody, for the manufacture of a medicament for the treatment of cancer.

The present invention also relates to a pharmaceutical composition for use in treating cancer comprising as an active ingredient a recombinant, isolated toxin-free antibody capable of specifically binding an MHC class I-peptide complex in cancer cells specifically expressing said MHC class I-peptide complex, wherein said recombinant, isolated toxin-free antibody is selected from the group consisting of a Fab fragment and a single chain antibody, said recombinant, isolated toxin-free antibody being capable of inducing apoptosis in said cancer cells and a pharmaceutically acceptable carrier.

Preferably the method further comprises detecting apoptosis in said cancer cells following said administering or said expressing.

Preferably the method detecting apoptosis is effected by a method selected from the group consisting of propidium iodide FACS analysis, Annexin V FACS analysis, Ethidium homodimer-1 staining, live/dead viability/cytotoxicity assay and Tunnel assay.

Preferably the present invention comprises a single chain antibody is 9H set forth in SEQ ID NO:1, G1 set forth in SEQ ID NO:2 or CLA12 set forth in SEQ ID NO:17.

Preferably the present invention comprising said peptide is derived from a polypeptide selected from the group consisting of MUC1, gp100, HTERT, TAX, MART 1.

Preferably the recombinant antibody of the present invention comprises the six CDRs set forth by SEQ ID NOs:5-10, 11-16 or 19-24.

Preferably said cancer is melanoma or breast cancer.

Preferably said cancer is melanoma and said peptide is derived from tyrosinase.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

### In the drawings:

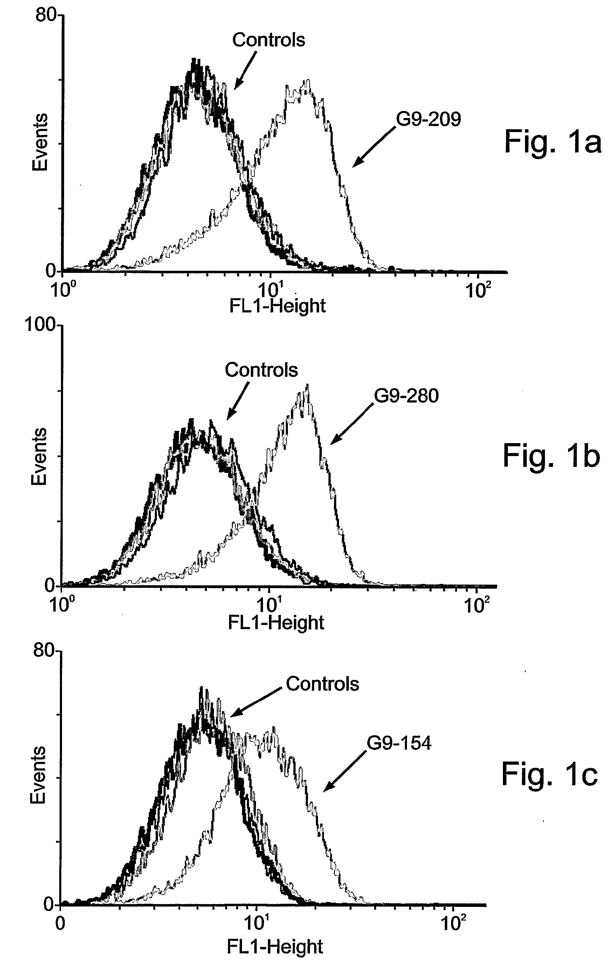

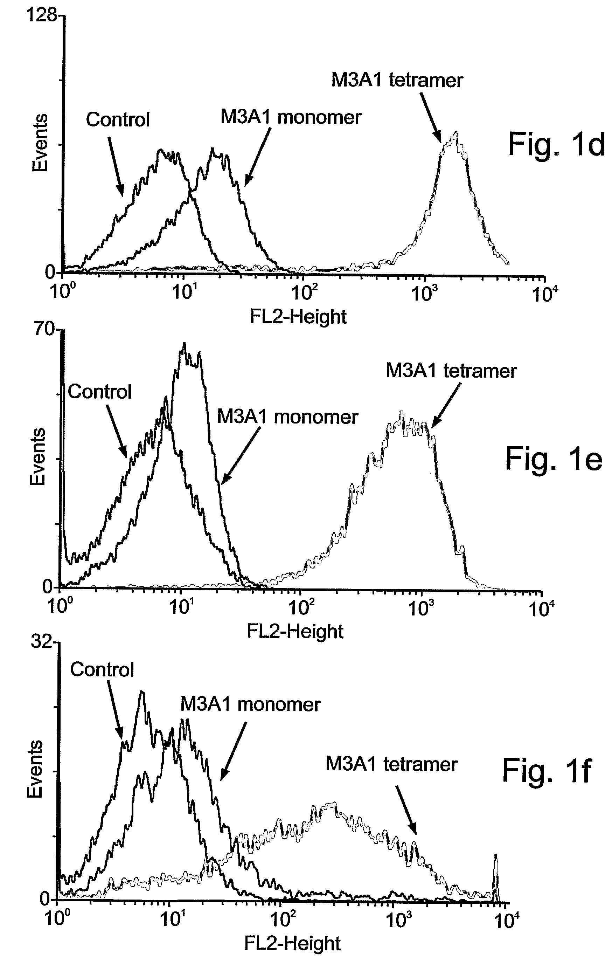

FIGs. 1a-f depict the binding of TCR-like antibodies to antigen-presenting cells. Figures 1a-c - RMAS-HHD cells were loaded with the G9-209 (Figure 1a), G9-280 (Figure 1b) and G9-154 (Figure 1c) melanoma differentiation antigen gp100-derived HLA-A2-restricted peptides, or with control HLA-A2 peptides (Figures 1a-c). Peptide loaded cells were incubated with the specific soluble purified ScFv antibodies: ScFv 1A7 (specific to G9-209; Figure 1a), ScFv 2F1 (specific to G9-280) and Fab G2D12 (specific to G9-154). Antibody binding was detected using a FITC-labeled anti-human Fab fragment. Figures 1d-f - Detection of HLA-A2/MUC1-D6 (MUC = mucin) complexes on the surface of cells using a Fab M3A1 tetramer. JY cells (Figure 1d), MDA-MB-231 breast carcinoma cells (Figure 1e), or HLA-A2⁺ mature dendritic cells (DC; Figure 1f) were pulsed with the mucine-derived MUC1-D6 HLA-A2-restricted peptide. Peptide pulsed-cells were then incubated with the HLA-A2/MUC1-D6-specific, pseudomonas exotoxin (PE)-labeled M3A1-tetramer or monomer antibodies. Fab monomer binding was detected with PE-labeled anti-human Fab. Control = unloaded cells, stained with the M3A1 tetramer.

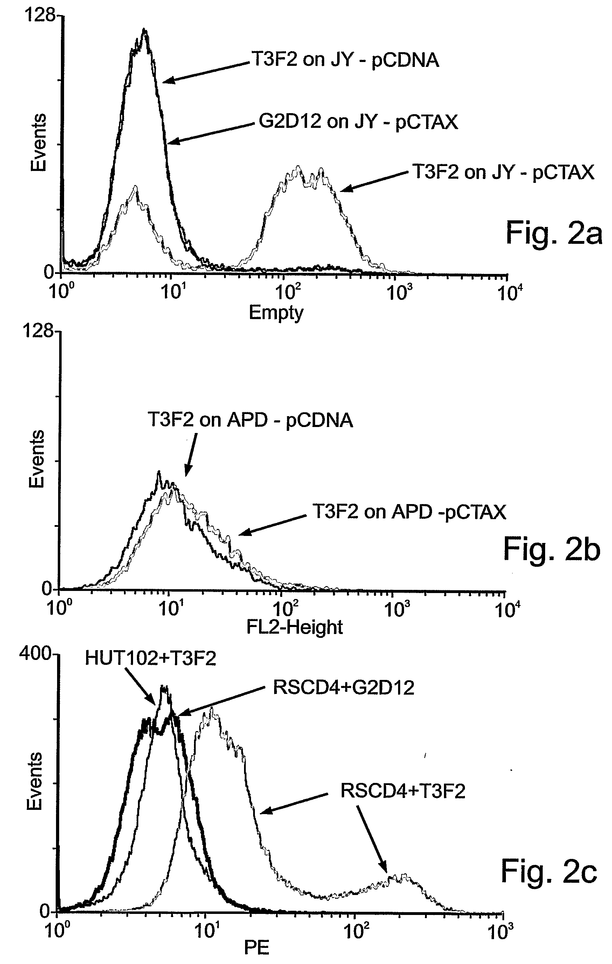

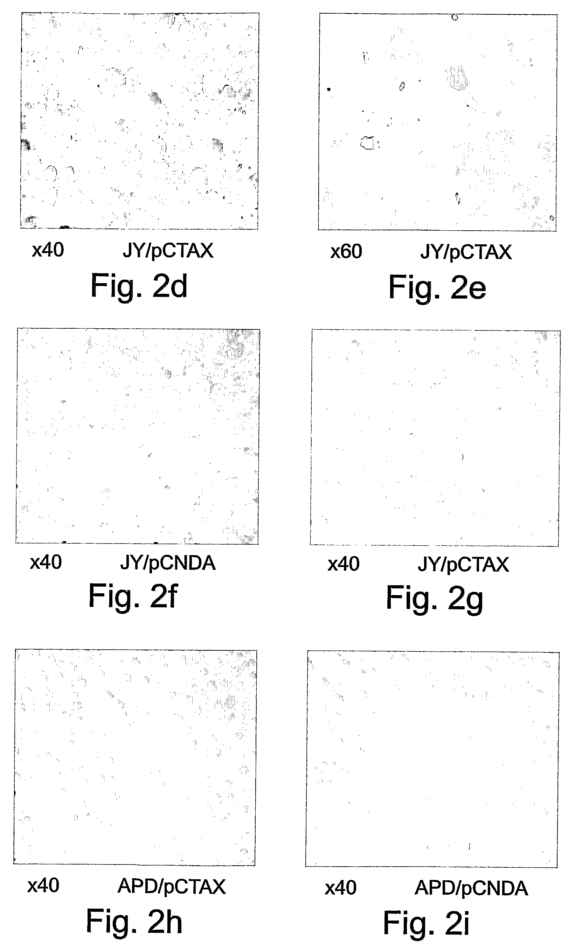

FIGs. 2a-i depict the detection of specific HLA-A2/peptide complexes by TCR-like antibodies after naturally occurring active intracellular processing. JY HLA-A2+ (Figure 2a) or APD HLA-A2- (Figure 2b) APCs were transfected with pCDNA control vector or with pCDNA containing the intact full length HTLV-1-derived Tax gene (pCTAX). Processing of this viral transcription factor gene was shown to result in the generation of the HLA-A2-restricted peptide epitope Tax₁₁₋₁₉. Twelve to twenty-four hours following transfection, cells were stained by flow cytometry using the HLA-A2/Tax₁₁₋₁₉-specific Fab T3F2 or a control G2D12 TCR-like Fab specific for melanoma differentiation antigen gp100 epitope, G9-154. The efficiency of Tax gene transduction into JY and APD cells was monitored by transfection of the pCDNA vector carrying the GFP gene (not shown). Figure 2c - HLA-A2⁺ RSCD4 and HLA-A2⁻ HUT102 cells, which are lines of human CD4+ T cells infected with HTLV-1, were stained with PE-labeled Fab tetramer T3F2 and control G2D12 as indicated. Figures 2d-i - Immunohistochemical staining of HLA-A2/Tax complexes after active intracellular processing. JY HLA-A2+ or HLA-A2-APD APCs were transfected with a pCDNA expression vector containing the Tax gene (Figures 2d, e, g, h) or with the expression vector alone (Figures 2f, i). Twelve to twenty-four hours following transfection, cells were absorbed onto poly-L-lysine coated glass slips and stained with the HLA-A2/Tax-specific Fab T3F2 (Figures 2d, e, f, h, i) or with the TCR-like Fab G2D12 specific for the melanoma gp100 HLA-A2/G9-154 epitope (Figure 2g). Figure 2e is a magnification (x 60) of the image presented in Figure 2d (x 40).

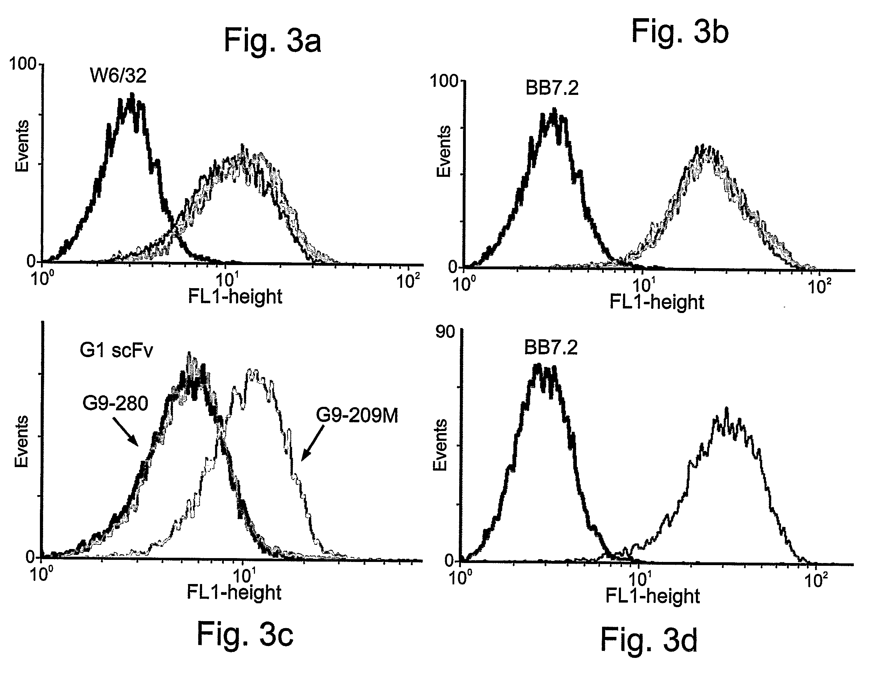

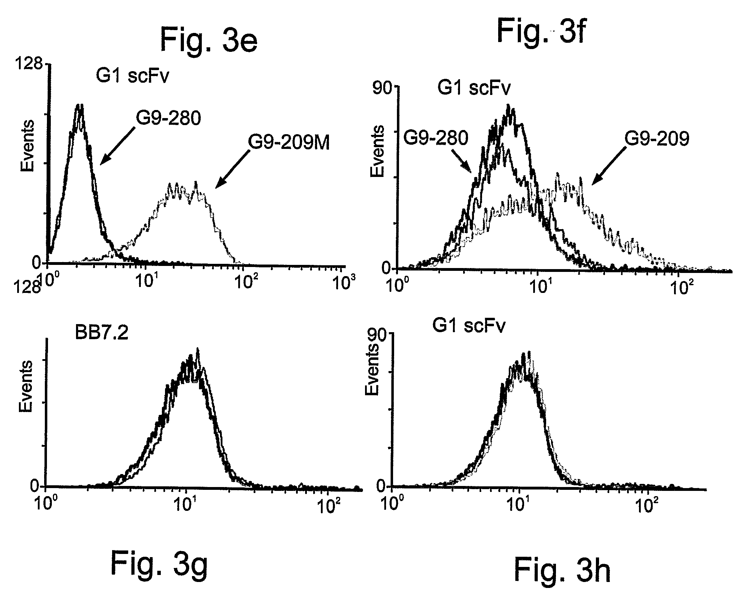

FIGs. 3a-h depict the binding of g1 scfv to peptide pulsed JY APCs (figures 3a-c), melanoma FM3D cells (figures 3d-f) or kb3-1 cells (figures 3g-h) cells, as determined by flow cytometry. Cells were pulsed for 3 hours at 37 °C with G9-280, G9-209, G9-209M or Tax HLA-A2-restricted peptides as indicated. Black = unloaded cells. Figures 3a-c - Cells were loaded with the G9-209M and G9-280 peptides and incubated with the soluble purified G1 scFv antibody (Figure 3c), the anti-HLA antibody W6/32 (Figure 3a), or the anti-HLA-A2 antibody BB7.2 (Figure 3b). Bound antibodies were detected using an FITC-labeled anti-Myc antibody. Note that while both the W6/32 and BB7.2 antibodies detected the HLA-peptide complexes on the cell surface (Figures 3a and b), the G1 antibody specifically detected the HLA-G9-209M complex but not the HLA-G9-280 complex (Figure 3c). Figures 3d-f - melanoma FM3D cells were pulsed with the G9-209M (Figure 3e), G9-209 (Figure 3f) or the control G9-280 (Figures 3d, e and f) gp100-derived peptides and subsequently stained with the BB7.2 anti-HLA-A2 (Figure 3d) or the G1 scFv (Figures 3e, f) antibodies. Note that while the BB7.2 antibody detected cells which were pulsed with the G9-280 control peptide (Figure 3d), the G1 antibody specifically detected cells which were pulsed with the G9-209 (Figure 3f) or the G9-209M (Figure 3e). Also note that the fraction of G1-positive cells loaded with the mutant G9-209M peptide was significantly higher than that of cells loaded with the G9-209 peptide. Figures 3g-h - HLA-A2 negative KB3-1 cells were pulsed with the G9-209M or G9-280 gp100-derived peptides and were stained with the BB7.2 (Figure 3g) or the G1 scFv (Figure 3h) antibodies. Note the absence of stained cells in cells lacking endogenous HLA-A2 molecules.

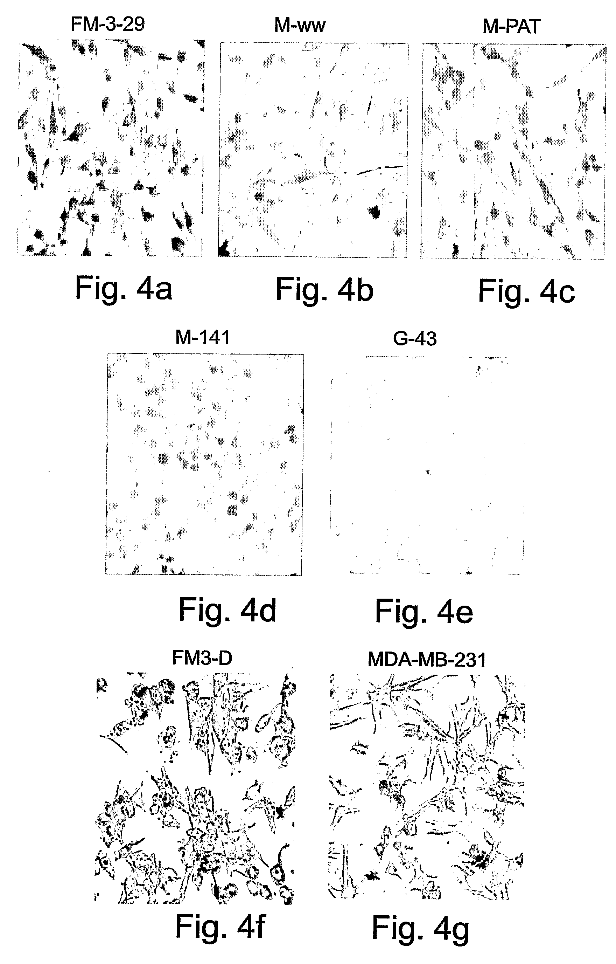

FIGs. 4a-g are immunohistochemical analysis depicting staining of melanoma cells with the G1 scFv antibody. HLA-A2-positive (FM-3-29, M-ww, M-PAT, M-141, FM3-D; Figures 4a-d and f) or negative (G-43; Figures 4e), gp100 expressing-melanoma cells or control breast carcinoma cells (MDA-MB-231; Figures 4g) were stained with the G1 scFv antibody. The cells (5 x 10⁶) were incubated for 1 hour on ice with 20-30 µg G1 scFv in RPMI culture medium containing 10 % fetal calf serum (FCS) followed by a 45-minutes incubation on ice with an HRP-anti-Myc antibody. The cell suspension was applied onto glass slides pre-coated with 0.1 % Poly-L-Lysine (Sigma) as described (Harlow and Lane, 1988). Cells were then incubated for 1 hour at room temperature, washed 3 times with phosphate buffer saline (PBS), and incubated for 1 minute with a DAB+ solution (Dako), following which the excess of the staining reagent was washed off with PBS. Cell nuclei were stained with hematoxylin (Sigma). FM3-D, FM-3-29, M-ww, M-PAT, M-141 and G-43 are melanoma cell lines derived from melanoma patients; MDA-MB-231 is a cell line derived from a breast carcinoma patient.

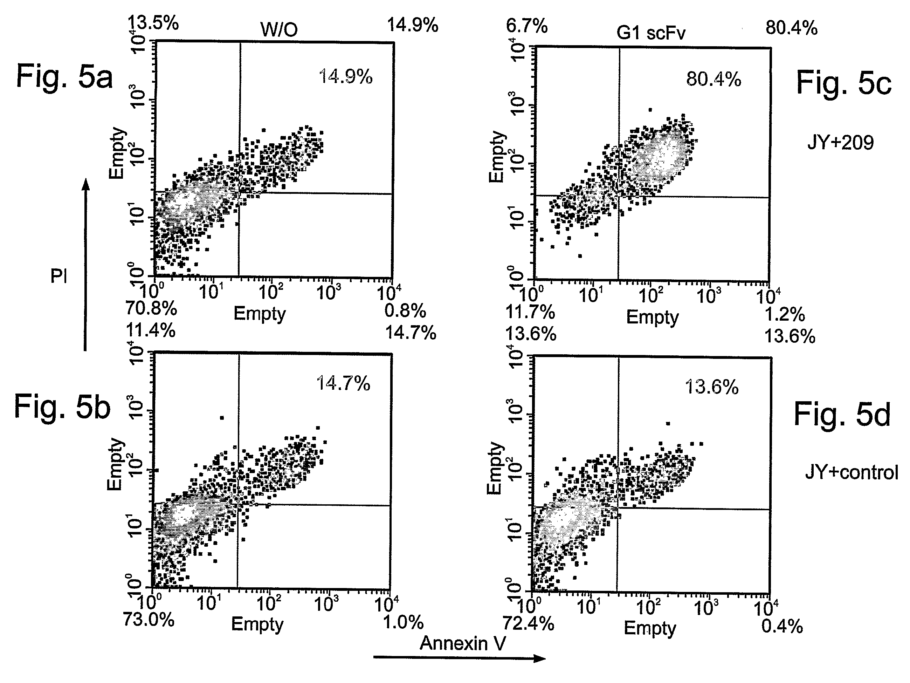

FIGs. 5a-d are FACS analyses depicting cytotoxicity of G1 scFv to peptide-pulsed APCs. RMAS-HHD (not shown) and JY APCs were loaded with the G9-209 or the control Tax peptides. Peptide-loaded cells were subsequently incubated with 25-50 µg of G1 scFv (Figures 5c and d) or remained untreated (Figures 5a and b). The cells were stained with anti-Annexin V (a marker for early apoptosis) and Propidium iodine (PI) and were subject to flow cytometry. Note the significant increase in Annexin V-labeled cells in the presence of the G1 scFv antibody in cells loaded with the G9-209 peptide (Figure 5c) as compared with cells loaded with the control Tax peptide (figure 5d). Also note that in the absence of the G1 scFv antibody, no difference in Annexin C labeling is observed between cells loaded with G9-209 (Figure 5a) and cells loaded with the control Tax peptide.

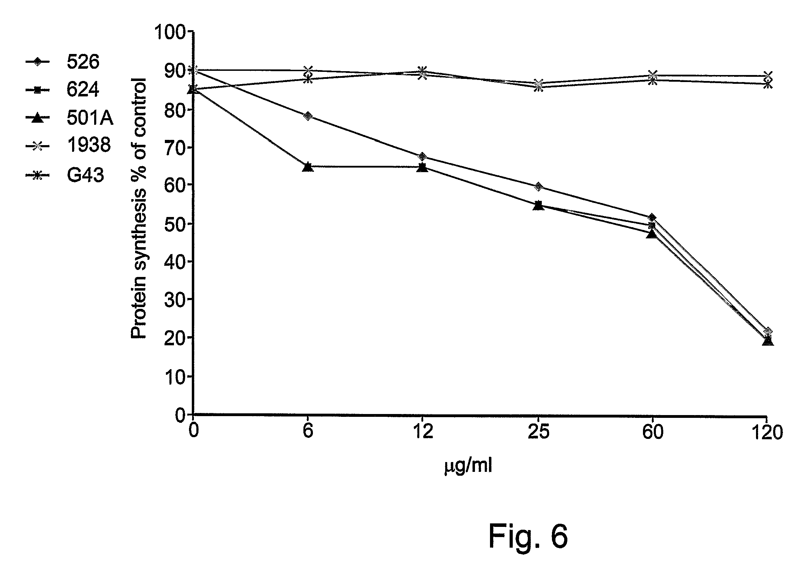

FIG. 6 is a graph depicting cytotoxicity assays with the G1 scFv antibody on melanoma cells. Various melanoma cell-lines [HLA-A2+/gp100+ cells (526, 501, 624), HLA-A2-/gp100+ cells (G-43) and HLA-A2+/gp100- cells (1938)] were incubated with increasing concentrations of the G1 scFv antibody and the inhibition of protein synthesis was determined by measuring the uptake of ³H-Leucine into cellular proteins. IC₅₀ was determined as the concentration of G1 scFv required to inhibit protein synthesis by 50 %. IC₅₀ was found to be 60□ µg/ml for the 526, 624 and 501A melanoma cell-lines. These results demonstrate that TCR-like antibodies can induce apoptosis in melanoma target cells.

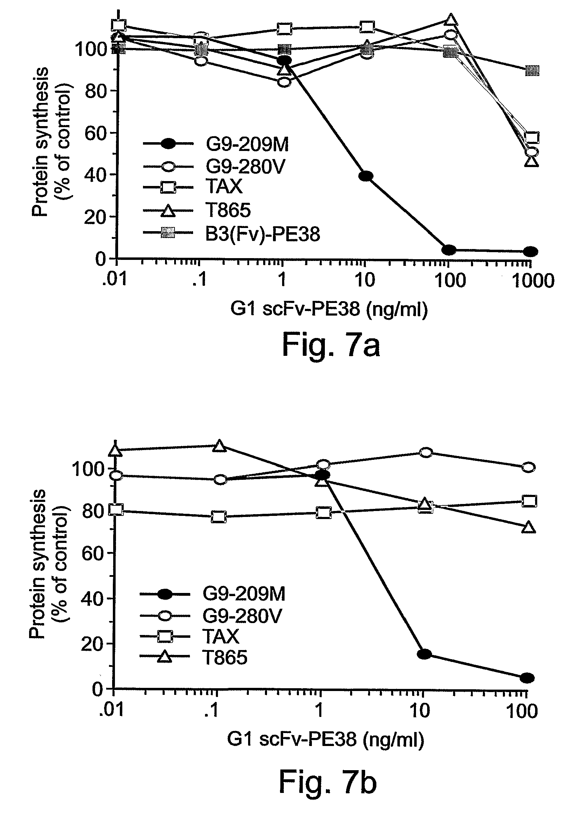

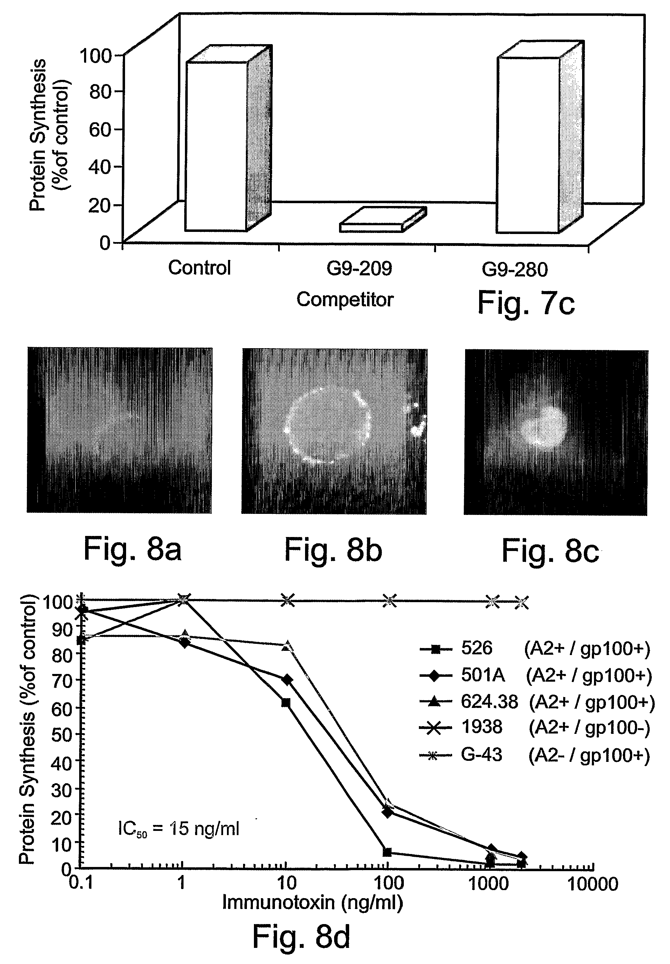

FIGs. 7a-c are graphs depicting cytotoxic activity of G1 scFv-PE38 on peptide-loaded APCs. The TCR-like antibody G1 was fused to a truncated version of pseudomonas exotoxin (PE) A, PE38. RMAS-HHD (Figure 7a) or JY (Figure 7b) cells were loaded with the G9-209M and G9-280V HLA-A2-restricted peptides derived from gp100, T865 peptide (derived from the telomerase catalytic subunit), TAX peptide (derived from the HTLV-1 Tax protein) or B3(Fv)-PE38 (a control non-specific immunotoxin directed to the Lewis Y antigen). Peptide loaded cells were incubated with increasing concentrations of G1 scFv-PE38. Protein synthesis was determined by incorporation of ³H-Leucine into cellular proteins. Note the specific toxicity observed in cells loaded with the G9-209M peptide in the presence of the specific HLA-G9-209 complex. Figure 7c - excess (0.15-0.25 mg/ml) of the indicated scHLA-A2-peptide complex is added to wells prior to the addition of G1 scFv-PE38 (25-50 ng/ml). In the control, no MHC-peptide complex was added. Note the complete inhibition of protein synthesis in cells expressing the scHLA-A2-G9-209 complex.

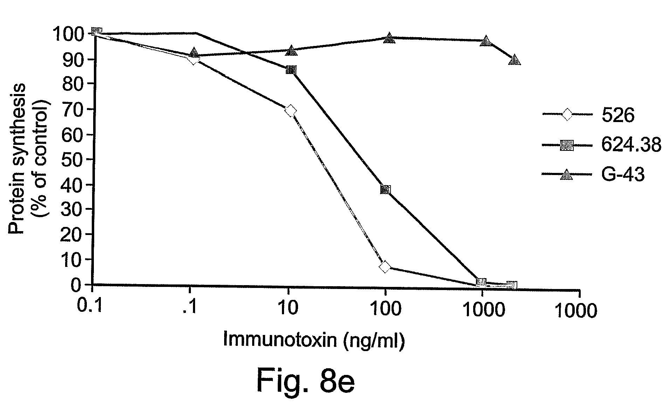

FIGs. 8a-e are immunostaining (Figures 8a-c) and graphs (Figures 8d-e) depicting internalization of TCR-like antibodies (Figures 8a-c) and killing of melanoma cells (Figures 8d-e). Figures 8a-c - TCR-like antibody 2F1 scFv targeting gp100 280 epitope was fused to a truncated version of pseudomonas exotoxin (PE) A, PE38, and was labeled with FITC. Fluorescence was followed by confocal microscopy at time 0 (Figure 8a), 15 minutes (Figure 8b), and 360 minutes (figure 8c) at 37 °C. Capping was observed after 15 minutes and after 360 minutes significant fluorescence was observed in the cytosol indicative of efficient internalization. Figures 8d-e - graphs depicting the effect of 2F1 directed to gp100 (Figure 8d) or CLA12 scFv directed to a MART1-derived HLA-A2/peptide complex (Figure 8e) on protein synthesis (followed by measuring incorporation of ³H-Leucine into cellular proteins) of melanoma cell lines derived from various patients. Inhibition of protein synthesis was calculated as a percentage of cells which were not subject to antibody treatment. Note that while all HLA-A2+/gp100+ melanoma cells (526, 501A, 624.38) were sensitive to the 2F1 cytotoxicity, the HLA-A2-/gp100+ (G-43) or HLA-A2+/gp100- (1938) melanoma cell-lines were resistant to antibody treatment (Figure 8d). For comparison, note the effect of the CLA12 antibody (SEQ ID NO:17) on inhibition of cell synthesis of 624-38 and 526 cells but not of G-43 (Figure 8e).

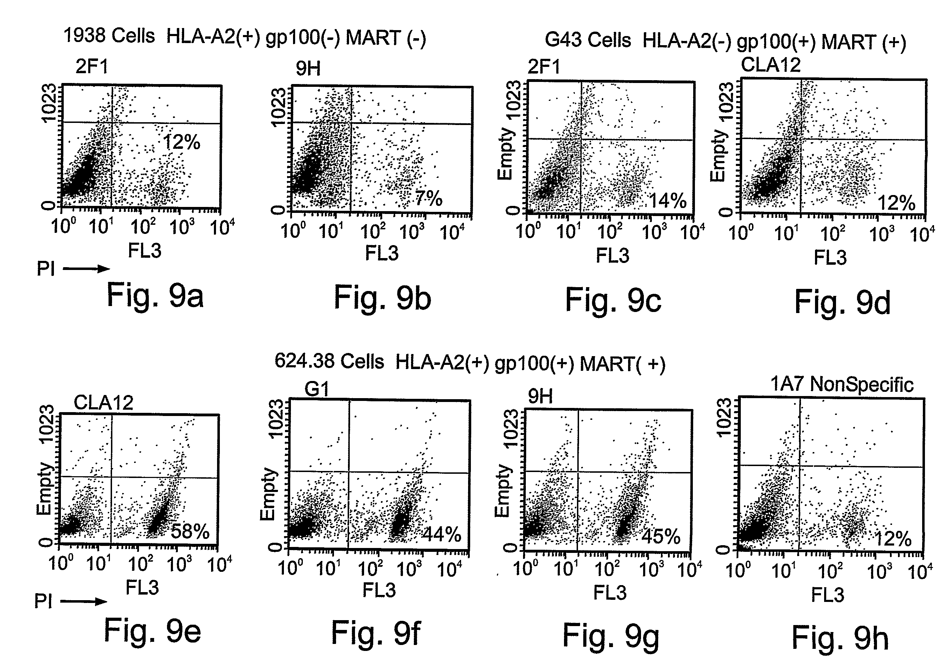

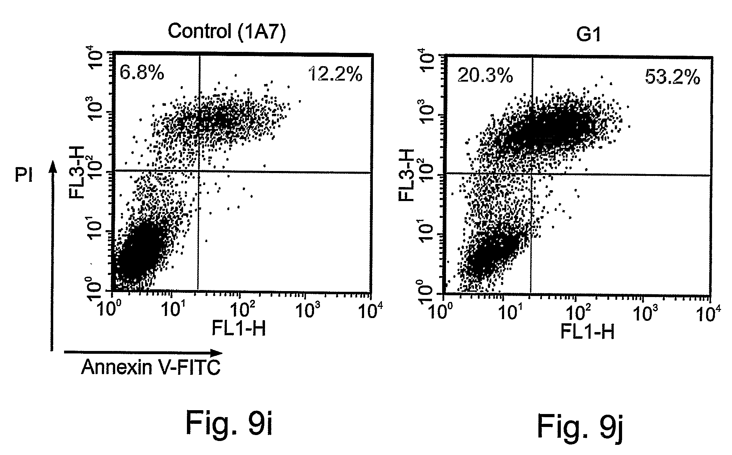

FIGs. 9a-j are FACS analyses depicting cell death of melanoma cells induced by the naked (toxin-free) TCR-like antibody scFv fragment. Figures 9a-h - Cells which are HLA-A2+/antigenic peptide+ (Figures 9e-h), HLA-A2-/antigenic peptide+ (Figures 9c-d) or HLA-A2+/antigenic peptide- (Figures 9a-b) were treated with the 2F1 (Figures 9a and c), 9H (figures 9b and g), and G1 (Figure 9f) naked TCR-like antibody fragments directed to gp100 derived peptides, the CLA12 naked TCR-like antibody (Figures 9d and e) directed against the MART1 derived peptide or with the 1A7 control antibody (which is specific against the HLA-A2-G9-209M but does not recognize the HLA-A2-G9-209 complex). Measurement of cell death was effected via Propidium Iodine (PI) flow cytometry staining and the percentages of PI positive cells is indicated. Figures 9i-j - Melanoma cells (624) which are gp100+/HLA-A2+ were incubated with the G1 (which is specific against the HLA-A2-G9-209 complex) (Figure 9j) or the control 1A7 (Figure 9i) antibodies. Measurement of cell death by Propidium Iodine (PI) and Annexin V flow cytometry staining and the percentages of positive cells are indicated.

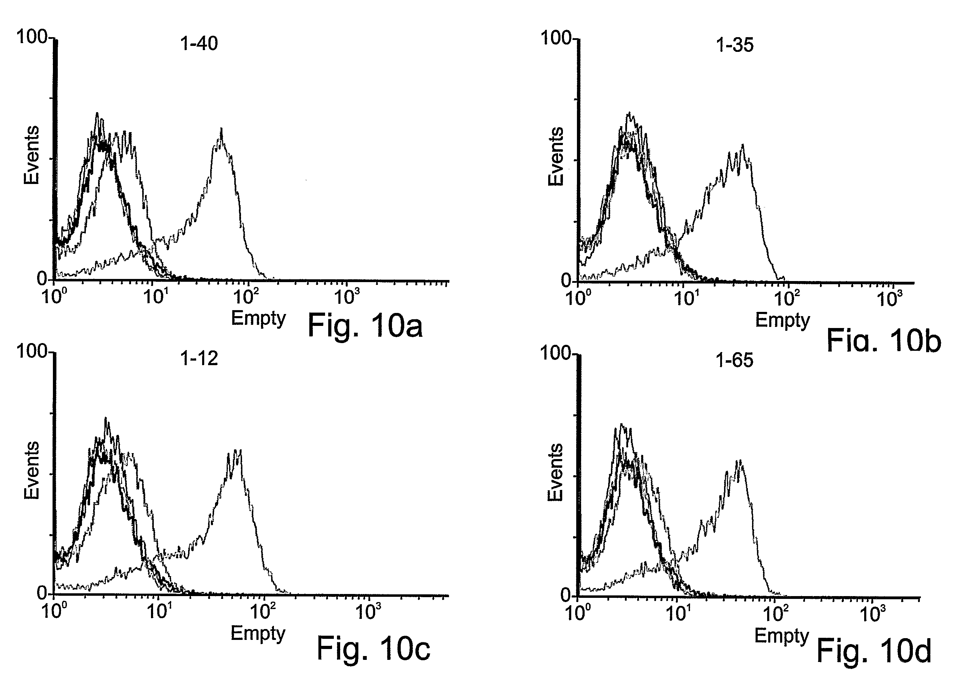

FIGs. 10a-d are flow cytometry analyses depicting transformation of TCR-like antibody ScFv fragments into whole IgG molecule and the induction of cell death in melanoma cells. TCR-like antibody 9H targeting gp100 HLA-A2/G9-209 peptide was transformed into a whole IgG molecule and its reactivity on peptide-pulsed JY cells was tested (Figures 10a-d). Four different transfected clones are shown: 1-40 (Figure 10a), 1-35 (Figure 10b), 1-12 (Figure 10c) and 1-65 (Figure 10d). Red -G9-209, green - EBV, blue - Muc1, purple - Tax, black - CMV. Controls are various HLA-A2 restricted peptides (Tax, EBV, Muc1, CMV).

### DESCRIPTION OF THE PREFERRED EMBODIMENTS

The present disclosure is a method of inducing apoptosis in cells expressing an MHC-peptide complex in a peptide-specific, MHC-restricted manner. Specifically, the present disclosure can be used to diagnose and treat cancer or pathogen infection using naked, toxin-free and highly potent antibodies capable of specifically binding MHC-peptide complexes with an affinity at the nanomolar range.

The principles and operation of the method of treating and diagnosing cancer or pathogen infection according to the present invention may be better understood with reference to the drawings and accompanying descriptions.

Before explaining at least one embodiment of the invention in detail, it is to be understood that the invention is not limited in its application to the details set forth in the following description or exemplified by the Examples. The invention is capable of other embodiments or of being practiced or carried out in various ways. Also, it is to be understood that the phraseology and terminology employed herein is for the purpose of description and should not be regarded as limiting.

The major histocompatibility complex class I (MHC-I) plays a central role in human immune system, by acting as a framework for peptide presentation to CD81 T-cells (Boon T. van der B.P., 1996). The peptides presented by the MHC complex (HLA-A-β2 microglobulin) are degradation products of various endogenously expressed proteins, exogenous proteins (e.g., which are derived from pathogens such as viruses or bacteria) and/or cancer-related proteins (e.g., the gp100 melanoma differentiation protein) which are obtained via the proteasome. The MHC-peptide complex is then recognized as an antigen by the cytotoxic T lymphocytes [Klein and Sato, 2000].

To study MHC-peptide presentation on the surface of macrophages, dendritic cells, tumor cells or virus-infected cells, soluble T-cell receptors (TCRs) were prepared in *E. coli.* However, such TCRs were instable and exhibited low affinity for their ligands [Wulfing and Pluckthun., 1994]. Several studies attempted to prepare TCR-like antibodies in murine systems [Murphy et. al., 1989; Aharoni et. al., 1991; Andersen et. al., 1996; Reiter et. al., 1997; Porgador et. al., 1997; Day et. al., 1997; Dadaglio et. al., 1997; Zhong et. al., 1997a; Zhong et. al., 1997b; Krogsgaard et. al., 2000; Polakova et. al., 2000]. However, antibodies with an exquisite of T-cell receptor-like specificity have proven difficult to produce using conventional hybridoma techniques or immunization strategies even in combination with *in vitro* selection from phage display libraries. The direct selection of such antibodies from very large non-immune phage antibody libraries was only recently demonstrated [Chames et. al., 2000; Supra]. Using this route, a phage clone (G8) which recognizes the melanoma antigen MAGE-A1 in complex with the human HLA-A1 MHC molecule was isolated. However, the affinity of the G8 phage towards the MHC-peptide complex was relatively low (250 nM). To overcome the low affinity limitation, Chames P., et al. (2002; J. Immunol. 169:1110-8) engrafted T cells with the *in vitro* engineered G8 antibody and prepared cell-specific antibodies. However, such antibodies are impractical for treating cancer or pathogen infection since they require gene therapy manipulations on a patient-specific basis.

The present inventors have previously isolated the G1 TCR like antibody from a murine scFv phage display library constructed from HLA-A2 transgenic mice which were immunized with the stable single-chain HLA-A2-β2 molecule complexed with the G9-209 melanoma-specific peptide (Denkberg G., et al., The Journal of Immunology, 2003; 171(5):2197-207). In contrast to prior art attempts, this scFv recombinant antibody (G1), which was isolated and soluble (*i.e*., not as a phage clone), exhibited a high affinity (within the nanomolar range, e.g., 5 nM) to the HLA-A2-G9-209 complex, in a peptide specific, MHC-restricted manner, similar to the specificity of TCRs (Denkberg G., 2003, Supra).

In sharp contrast to prior arts, the present inventors have uncovered that TCR-like recombinant isolated antibodies (*i.e*., cell-free), which specifically bind MHC-peptide complexes with an affinity in the nanomolar range (e.g., 5 nM), are capable of inducing apoptosis in cells expressing such complexes. In addition, the present inventors have uncovered new recombinant and isolated scFv antibodies [clones 9H (SEQ ID NO:1) and CLA12 (SEQ ID NO:17)] from a large human synthetic single-chain Fv antibody library [Azriel-Rosenfeld R, Valensi M, Benhar I. A human synthetic combinatorial library of arrayable single-chain antibodies based on shuffling in vivo formed CDRs into general framework regions. J Mol Biol. 2004 Jan 2; 335(1):177-92]. These antibodies are capable of specifically binding the HLA-A2-G9-209 complex (scFv 9H) or the HLA-A2-MART1 complex (scFv CLA12) with an affinity of 0.5 nM (for scFv 9H) or 10 nM (for scFv CLA12).

Thus, as is shown in Figures 3a-h, 5a-d, 6 and 9a-j and the Examples section which follows, the previously isolated G1 antibody (Denkberg et. al., 2003, Supra), the 9H (SEQ ID NO:1) and the CLA12 (SEQ ID NO:17) antibodies can be used as naked, toxin-free, potent agents for the selective induction of apoptosis of cells expressing the specific MHC-peptide complexes (e.g., cancer cells) in a peptide-specific, HLA-restricted manner.

Thus, according to one aspect of the present disclosure, there is provided a method of inducing apoptosis in cancer cells or pathogen infected cells. The method is effected by contacting, or expressing in the cells a recombinant isolated antibody capable of specifically binding an MHC-peptide complex specifically expressed on the cells, thereby inducing apoptosis in the cancer cells or the pathogen infected cells.

As used herein the term "apoptosis" refers to programmed cell death whereby the cell executes a "cell suicide" program. Apoptosis plays an important role in a number of physiological events including embryogenesis, regulation of the immune system, and homeostasis. Thus, apoptosis can be in response to diverse signals such as limb and neural development, neurodegenerative diseases, radiotherapy and chemotherapy. Apoptotic processes are usually characterized by uncoupling of mitochondrial oxidation, drop in levels of nicotinamide adenine dinucleotide phosphate [NAD(P)H], release of cytochrome c, activation of caspases, DNA fragmentation and externalization of phosphatidylserine (a membrane phospholipid normally restricted to the inner leaflet of the lipid bilayer) to the outer leaflet of the plasma membrane.

As used herein the phrase MHC-peptide complex refers to a protein complex formed between any MHC molecule (or complex) and a peptide. The term MHC encompasses several classes of MHC complexes (e.g., MHC class I, II, III and the like). According to one preferred embodiment of the present disclosure, the term MHC refers to MHC-class I. The MHC-class I molecules are expressed on the surfaces of most cells and are recognized by CD8-positive cytotoxic TC-cells. MHC class I molecules are heterodimers composed of α and a β subunits. The α subunit includes three structural domains (α1, α2, α3) and is encoded by the MHC gene cluster (e.g., the HLA-A1 or HLA-A2 genes). The α1 and α2 structural domains associate to form the peptide-binding pocket and the membrane-distal region of the molecule. The third structural domain of the α subunit - α3 - noncovalently associates with the β subunit β2 microglobulin; β2m), forming the membrane-proximal region of the molecule. The β2m is not encoded by the MHC gene cluster.

The peptide which is bound to the MHC molecule can be any peptide obtained by proteasomal degradation of a protein. Such a protein can be of a pathogenic protein, which is derived from the pathogen [e.g., bacteria, viruses, fungi and the like], as well as a protein which is usually not expressed in normal cells, but rather is uniquely expressed in cancerous or pre-cancerous cells (e.g., tumor promoter proteins, tumor differentiation proteins and the like). Non-limiting examples of MHC-peptide complexes include the HLA-A2-G9-209, HLA-A2-G9-280 and HLA-A2-MART1. Further examples are provided in the Examples section which follows.

Preferably, the peptide of the MHC-peptide complex is derived from a protein such as MUC1 (see Cohen et al., 2002), gp100 (GenBank Accession No. AAB31176), HTERT (GenBank Accession No. AAD30037), TAX (see Cohen et al., 2003), and MART1 (GenBank Accession No. Q16655).

According to the method of the present disclosure, the recombinant isolated antibody is capable of specifically binding an MHC-peptide complex specifically expressed on the cells. For example, as is shown in Figures 3a-h, cells pulsed with an antigenic peptide were specifically labeled with the G1 antibody only when expressing the specific HLA-A2 allele. In addition, as is shown in Figures 4a-g, the G1 TCR-like antibody specifically recognized natural non-pulsed melanoma cells expressing the gp100-derived G9-209 peptide.

The term " recombinant antibody" as used in this disclosureincludes intact molecules as well as functional fragments thereof, such as Fab, F(ab')2, Fv or single domain molecules such as VH and VL to an epitope of an antigen at least partially generated by recombinant DNA technology. These functional antibody fragments are defined as follows: (1) Fab, the fragment which contains a monovalent antigen-binding fragment of an antibody molecule, can be produced by digestion of whole antibody with the enzyme papain to yield an intact light chain and a portion of one heavy chain; (2) Fab', the fragment of an antibody molecule that can be obtained by treating whole antibody with pepsin, followed by reduction, to yield an intact light chain and a portion of the heavy chain; two Fab' fragments are obtained per antibody molecule; (3) (Fab')2, the fragment of the antibody that can be obtained by treating whole antibody with the enzyme pepsin without subsequent reduction; F(ab')2 is a dimer of two Fab' fragments held together by two disulfide bonds; (4) Fv, defined as a genetically engineered fragment containing the variable region of the light chain and the variable region of the heavy chain expressed as two chains; (5) Single chain antibody ("SCA"), a genetically engineered molecule containing the variable region of the light chain and the variable region of the heavy chain, linked by a suitable polypeptide linker as a genetically fused single chain molecule (scFv); and (6) Single domain antibodies are composed of a single VH or VL domains which exhibit sufficient affinity to the antigen.

The term "antibody" as used herein is not only inclusive of antibodies generated by immunization and recombinant phage display techniques, but also includes any polypeptide which is generated to include at least one complementary-determining region (CDR) which is capable of specifically binding to the MHC-peptide complex of the present disclosure (see for example, Azriel-Rosenfeld R, et al., 2004; J Mol Biol. 335(1):177-92). Thus, the antibody of the present disclosurecan be expressed (as is further described hereinbelow) from a polynucleotide sequence including a coding sequence of at least one CDR of an antibody.

To increase avidity of the antibody, towards the MHC-peptide complex, antibodies of the present disclosureare preferably at leasr bivalent. Such antibodies can be cloned into IgG subtype (which is bivalent), IgM (which is penta-valent) or selected of such subtypes using methods known in the arts. Alternatievly, antibodies can be chemically conjugated such as by using a cross-linker or a sequence tag (e.g., BirA, see Example 1 of the Examples section which follows).

Methods of producing polyclonal and monoclonal antibodies as well as fragments thereof are well known in the art (See for example, Harlow and Lane, Antibodies: A Laboratory Manual, Cold Spring Harbor Laboratory, New York, 1988).

Antibody fragments according to the present disclosurecan be prepared by proteolytic hydrolysis of the antibody or by expression in *E. coli* or mammalian cells (e.g. Chinese hamster ovary cell culture or other protein expression systems) of DNA encoding the fragment. Antibody fragments can be obtained by pepsin or papain digestion of whole antibodies by conventional methods. For example, antibody fragments can be produced by enzymatic cleavage of antibodies with pepsin to provide a 5S fragment denoted F(ab')2. This fragment can be further cleaved using a thiol reducing agent, and optionally a blocking group for the sulfhydryl groups resulting from cleavage of disulfide linkages, to produce 3.5S Fab' monovalent fragments. Alternatively, an enzymatic cleavage using pepsin produces two monovalent Fab' fragments and an Fc fragment directly. These methods are described, for example, by Goldenberg, U.S. Pat. Nos. 4,036,945 and 4,331,647, and references contained therein. See also Porter, R. R. [Biochem. J. 73: 119-126 (1959)]. Other methods of cleaving antibodies, such as separation of heavy chains to form monovalent light-heavy chain fragments, further cleavage of fragments, or other enzymatic, chemical, or genetic techniques may also be used, so long as the fragments bind to the antigen that is recognized by the intact antibody.

Fv fragments comprise an association of VH and VL chains. This association may be noncovalent, as described in Inbar et al. [Proc. Nat'1 Acad. Sci. USA 69:2659-62 (19720]. Alternatively, the variable chains can be linked by an intermolecular disulfide bond or cross-linked by chemicals such as glutaraldehyde. Preferably, the Fv fragments comprise VH and VL chains connected by a peptide linker. These single-chain antigen binding proteins (scFv) are prepared by constructing a structural gene comprising DNA sequences encoding the VH and VL domains connected by an oligonucleotide. The structural gene is inserted into an expression vector, which is subsequently introduced into a host cell such as *E. coli.* The recombinant host cells synthesize a single polypeptide chain with a linker peptide bridging the two V domains. Methods of producing scFvs are described, for example, by Whitlow and Filpula, Methods 2: 97-105 (1991); Bird et al., Science 242:423-426 (1988); Pack et al., Bio/Technology 11:1271-77 (1993); and U.S. Pat. No. 4,946,778.

Another form of an antibody fragment is a peptide coding for a single complementary-determining region (CDR). CDR peptides ("minimal recognition units") can be obtained by constructing genes encoding the CDR of an antibody of interest. Such genes are prepared, for example, by using the polymerase chain reaction to synthesize the variable region from RNA of antibody-producing cells. See, for example, Larrick and Fry [Methods, 2: 106-10 (1991)]. For example, such a peptide can be at least one peptide from the group of peptides set forth by SEQ ID NOs:5-10 (which are derived from the 9H scFv), SEQ ID NOs:11-16 (which are derived from the G1 scFv) or SEQ ID NOs:19-24 (which are derived from the CLA12 scFv).

Humanized forms of non-human (e.g., murine) antibodies are chimeric molecules of immunoglobulins, immunoglobulin chains or fragments thereof (such as Fv, Fab, Fab', F(ab')₂ or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the framework regions (FR) are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as import residues, which are typically taken from an import variable domain. Humanization can be essentially performed following the method of Winter and co-workers [Jones et al., Nature, 321:522-525 (1986); Riechmann et al., Nature 332:323-327 (1988); Verhoeyen et al., Science, 239:1534-1536 (1988)], by substituting rodent CDRs or CDR sequences for the corresponding sequences of a human antibody. Accordingly, such humanized antibodies are chimeric antibodies (U.S. Pat. No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter, J. Mol. Biol., 227:381 (1991); Marks et al., J. Mol. Biol., 222:581 (1991)]. The techniques of Cole et al. and Boerner et al. are also available for the preparation of human monoclonal antibodies (Cole et al., Monoclonal Antibodies and Cancer Therapy, Alan R. Liss, p. 77 (1985) and Boerner et al., J. Immunol., 147(1):86-95 (1991); Chames P., et al., 2000, Proc Natl Acad Sci USA. 97(14):7969-74; Azriel-Rosenfeld R, et al., 2004, J. Mol. Biol. 335(1): 177-92; Denkberg G., et al., 2001, J. Immunol., 167: 270-276; Denkberg J. Immunol, Benhar, I., and Reiter, Y., 2002, Phage display of single-chain antibody (scFv) constructs. Current Protocols in Immunology. 48:10.4 pp 59-87; Brekke OH, Loset GA. New technologies in therapeutic antibody development. Curr Opin Pharmacol. 2003, 3(5):544-50; Benhar I, Biotechnological applications of phage and cell display. Biotechnol Adv. 2001, 19(1):1-33]. Similarly, human antibodies can be made by introduction of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Pat. Nos. 5,545,807; 5,545,806; 5,569,825; 5,625,126; 5,633,425; 5,661,016, and in the following scientific publications: Marks et al., Bio/Technology 10,: 779-783 (1992); Lonberg et al., Nature 368: 856-859 (1994); Morrison, Nature 368 812-13 (1994); Fishwild et al., Nature Biotechnology 14, 845-51 (1996); Neuberger, Nature Biotechnology 14: 826 (1996); and Lonberg and Huszar, Intern. Rev. Immunol. 13, 65-93 (1995).

According to one preferred embodiment, the antibody of the present disclosureis isolated from a non-immune phage library essentially as described in Cohen et. al., 2002; Lev et. al., 2002; Denkberg et. al., 2002a, Cohen et al 2003.

According to preferred embodiments of the present disclosure, the recombinant isolated antibody is a recombinant polypeptide comprising at least one CDR sequence as set forth by SEQ ID NOs:5-10, 11-16 and/or 19-24. Examples include, but are not limited to the scFv fragment 9H (SEQ ID NO:1, G1 (SEQ ID NO:2) or CLA12 (SEQ ID NO:17)] and/or any IgG clone including at least one CDR or scFv sequence. Such a recombinant polypeptide can be expressed from a polynucleotide encoding such at least one CDR or scFv from a DNA construct as is further described hereinbelow.

It will be appreciated that in case an IgG antibody is produced (using recombinant DNA technologies known in the art and as is further described hereinbelow), such an IgG antibody can be further digested to produce a Fab fragment which is shorter and thus more soluble than the complete IgG antibody.

In addition, as is shown in Figures 7a-c, 8d-e and is described in the Examples section which follows, when the coding sequences of the G1, 2F1, or CLA12 scFvs were fused to coding sequence of the Pseudomonas exotoxin (PE38), a more potent, yet highly specific inhibition of protein synthesis was observed. Thus, using such immunotoxins, 50 % inhibition of protein synthesis in melanoma cells was achieved in the presence of only 10 ng/ml of the G1 scFv PE38 (Figure 7a), 15 ng/ml of the 2F1 scFv PE38 (Figure 8d) and 10-100 ng/ml of the CLA12 scFv PE38 (Figure 8e) immunotoxins. Moreover, as is further shown in Figures 7a, 8d and e, an almost complete inhibition of protein synthesis was achieved in the presence of 100 ng/ml of the specific immunotoxins. These results suggest the use of antibodies fused to toxins for the treatment of cancer cells or pathogen infected cells.

Thus, according to one preferred embodiment of present disclosure, the recombinant isolated antibody of the present disclosureis fused or conjugated to a cytotoxic agent to thereby form a specific immunotoxin. Such toxin agents can be, for example, the Pseudomonas exotoxins PE35, PE38, PE40, Pseudomonas aeroginosa exotoxin A (ETA'), diphtheria toxin (DT390), and the like. Additionally or alternatively, the recombinant isolated antibody of the presence disclosurecan be fused to or conjugated to a chemotherapy agent (e.g., Mechlorethamine, Fluorouracil, Dacarbazine, Docetaxel, Carmustine, Vindesine and the like), antibiotic agent or antiviral agent. It will be appreciated that such immunotoxins and/or chemotherapy agents can be generated using recombinant DNA techniques (e.g., by ligating the coding sequence of the agent molecule to the coding sequence of the recombinant antibody, usually downstream of the coding sequence of the recombinant antibody) or by covalently conjugating the toxin or chemotherapy agents to the recombinant isolated antibody polypeptide sequence (e.g., to the polypeptide set forth by SEQ ID NO: 1, 2, or 17) by methods known in the art. For example by using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bisazido compounds (such as bis-(p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a 5-FU peptide conjugate can be generated as described by Semko (1996) Peptides Abst. 24th Symp. Eur. Pept. Soc. P26. A ricin-peptide conjugate can be prepared as described in Vitetta et al., Science, 238: 1098 (1987). 1-isothiocyanatobenzyl-3-methyldiethylene triaminepentaacetic acid (MX-DTPA) is an exemplary chelating agent for conjugation of radionucleotide to the peptide (see WO94/11026).

The cancer cells in which apoptosis is induced can be derived from any kind of tumor, including solid tumors (e.g., breast cancer, colon cancer, lung cancer, melanoma) and non-solid tumors (e.g., leukemia and lymphoma). Preferably, the cancer cells of the present disclosureare breast cancer cells or melanoma cells. In addition, such cells can be derived by mutagenesis or transfection of normal cells with an oncogene using methods known in the art.

The pathogen infected cells in which apoptosis is induced can be any eukaryotic cells, preferably mammals, more preferably, human cells, which are infected with the pathogen, *i.e*., any virus, bacteria and/or fungi known in the arts. Such cells can be, for example, hematopoietic cells, lymphoid cells, dendritic cells, macrophages, skin cells, neurons, fibroblasts, endothelial cells, smooth muscle cells, and epithelial cells. The virus can be any virus which is capable of infecting the cells of the present disclosure. For example, human immunodeficiency virus (HIV), hepatitis A, B and C viruses, herpes virus, cytomegalovirus (CMV) and Rous sarcoma virus (RSV). The bacteria can be bacteria which is intracellular such as Listeria moncytogenes, Salmonella typhimurium, and Mycobacterium tuberculosis (Soloski MJ, et al., Proc Soc Exp Biol. Med. 2000, 224: 231-9).

According to the method of the present disclosure, the recombinant isolated antibody is contacted with or expressed in the cancer cells or pathogen infected cells of the present disclosure. Methods of administering antibodies to the cells are known in the art and include, for example, the addition of the antibody to the cell environment such as blood, plasma, buffers, tissue culture medium and the like. For example, as is shown in Figures 8a-c, the 2F1 antibody targeting gp100 280 epitope was added to the culture medium of the cells and was found inside the cells within 360 minutes.

Alternatively, the recombinant isolated antibody of the present disclosurecan be expressed in cells (e.g., mammalian cells, bacterial cells, plant cells, yeast cells) as part of a nucleic acid construct containing a polynucleotide encoding for example, the Fab fragment, the scFV, the complete IgG antibody or any of the CDRs of the recombinant isolated antibody of the present disclosure.

To express the recombinant isolated antibody of the present disclosurein mammalian cells, a polynucleotide sequence encoding at least one CDR sequence as set forth by SEQ ID NOs:5-16 and 19-24 is preferably ligated into a nucleic acid construct suitable for mammalian cell expression. Such a polynucleotide can be, for example, the polynucleotide set forth by SEQ ID NO:3 (for the 9H scFv), SEQ ID NO:4 (for the G1 scFv), SEQ ID NO:18 (for the CLA12 scFv). The nucleic acid construct includes a promoter sequence for directing transcription of the polynucleotide sequence in the cell in a constitutive or inducible manner.

Constitutive promoters suitable for use with the present disclosureare promoter sequences which are active under most environmental conditions and most types of cells such as the cytomegalovirus (CMV) and Rous sarcoma virus (RSV). Inducible promoters suitable for use with the present disclosureinclude for example the tetracycline-inducible promoter (Zabala M, et al., Cancer Res. 2004, 64(8): 2799-804).

The nucleic acid construct (also referred to herein as an "expression vector") of the present disclosureincludes additional sequences which render this vector suitable for replication and integration in prokaryotes, eukaryotes, or preferably both (e.g., shuttle vectors). In addition, typical cloning vectors may also contain a transcription and translation initiation sequence, transcription and translation terminator and a polyadenylation signal. By way of example, such constructs will typically include a 5' LTR, a tRNA binding site, a packaging signal, an origin of second-strand DNA synthesis, and a 3' LTR or a portion thereof.

The nucleic acid construct of the present disclosuretypically includes a signal sequence for secretion of the peptide from a host cell in which it is placed. Preferably the signal sequence for this purpose is a mammalian signal sequence or the signal sequence of the polypeptide variants of the present disclosure.

Eukaryotic promoters typically contain two types of recognition sequences, the TATA box and upstream promoter elements. The TATA box, located 25-30 base pairs upstream of the transcription initiation site, is thought to be involved in directing RNA polymerase to begin RNA synthesis. The other upstream promoter elements determine the rate at which transcription is initiated.

Preferably, the promoter utilized by the nucleic acid construct of the present disclosureis active in the specific cell population transformed. Examples of cell type-specific and/or tissue-specific promoters include promoters such as albumin that is liver specific [Pinkert et al., (1987) Genes Dev. 1:268-277], lymphoid specific promoters [Calame et al., (1988) Adv. Immunol. 43:235-275]; in particular promoters of T-cell receptors [Winoto et al., (1989) EMBO J. 8:729-733] and immunoglobulins; [Banerji et al. (1983) Cell 33729-740], neuron-specific promoters such as the neurofilament promoter [Byrne et al. (1989) Proc. Natl. Acad. Sci. USA 86:5473-5477], pancreas-specific promoters [Edlunch et al. (1985) Science 230:912-916] or mammary gland-specific promoters such as the milk whey promoter (U.S. Pat. No. 4,873,316 and European Application Publication No. 264,166).

Enhancer elements can stimulate transcription up to 1,000 fold from linked homologous or heterologous promoters. Enhancers are active when placed downstream or upstream from the transcription initiation site. Many enhancer elements derived from viruses have a broad host range and are active in a variety of tissues. For example, the SV40 early gene enhancer is suitable for many cell types. Other enhancer/promoter combinations that are suitable for the present disclosureinclude those derived from polyoma virus, human or murine cytomegalovirus (CMV), the long term repeat from various retroviruses such as murine leukemia virus, murine or Rous sarcoma virus and HIV. See, Enhancers and Eukaryotic Expression, Cold Spring Harbor Press, Cold Spring Harbor, N.Y. 1983.

In the construction of the expression vector, the promoter is preferably positioned approximately the same distance from the heterologous transcription start site as it is from the transcription start site in its natural setting. As is known in the art, however, some variation in this distance can be accommodated without loss of promoter function.

Polyadenylation sequences can also be added to the expression vector in order to increase the efficiency of recombinant isolated antibody mRNA translation. Two distinct sequence elements are required for accurate and efficient polyadenylation: GU or U rich sequences located downstream from the polyadenylation site and a highly conserved sequence of six nucleotides, AAUAAA, located 11-30 nucleotides upstream. Termination and polyadenylation signals that are suitable for the present disclosureinclude those derived from SV40.

In addition to the elements already described, the expression vector of the present disclosuremay typically contain other specialized elements intended to increase the level of expression of cloned nucleic acids or to facilitate the identification of cells that carry the recombinant DNA. For example, a number of animal viruses contain DNA sequences that promote the extra chromosomal replication of the viral genome in permissive cell types. Plasmids bearing these viral replicons are replicated episomally as long as the appropriate factors are provided by genes either carried on the plasmid or with the genome of the host cell.

The vector may or may not include a eukaryotic replicon. If a eukaryotic replicon is present, then the vector is amplifiable in eukaryotic cells using the appropriate selectable marker. If the vector does not comprise a eukaryotic replicon, no episomal amplification is possible. Instead, the recombinant DNA integrates into the genome of the engineered cell, where the promoter directs expression of the desired nucleic acid.

The expression vector of the present disclosurecan further include additional polynucleotide sequences that allow, for example, the translation of several proteins from a single mRNA such as an internal ribosome entry site (IRES) and sequences for genomic integration of the promoter-chimeric polypeptide.

Examples for mammalian expression vectors include, but are not limited to, pcDNA3, pcDNA3.1(+/-), pGL3, pZeoSV2(+/-), pSecTag2, pDisplay, pEF/myc/cyto, pCMV/myc/cyto, pCR3.1, pSinRep5, DH26S, DHBB, pNMT1, pNMT41, pNMT81, which are available from Invitrogen, pCI which is available from Promega, pMbac, pPbac, pBK-RSV and pBK-CMV which are available from Strategene, pTRES which is available from Clontech, and their derivatives.

Expression vectors containing regulatory elements from eukaryotic viruses such as retroviruses can be also used. SV40 vectors include pSVT7 and pMT2. Vectors derived from bovine papilloma virus include pBV-1MTHA, and vectors derived from Epstein Bar virus include pHEBO, and p2O5. Other exemplary vectors include pMSG, pAV009/A⁺, pMTO10/A⁺, pMAMneo-5, baculovirus pDSVE, and any other vector allowing expression of proteins under the direction of the SV-40 early promoter, SV-40 later promoter, metallothionein promoter, murine mammary tumor virus promoter, Rous sarcoma virus promoter, polyhedrin promoter, or other promoters shown effective for expression in eukaryotic cells.

As described above, viruses are very specialized infectious agents that have evolved, in many cases, to elude host defense mechanisms. Typically, viruses infect and propagate in specific cell types. The targeting specificity of viral vectors utilizes its natural specificity to specifically target predetermined cell types and thereby introduce a recombinant gene into the infected cell. Thus, the type of vector used by the present disclosurewill depend on the cell type transformed. The ability to select suitable vectors according to the cell type transformed is well within the capabilities of the ordinary skilled artisan and as such no general description of selection consideration is provided herein. For example, bone marrow cells can be targeted using the human T cell leukemia virus type I (HTLV-I) and kidney cells may be targeted using the heterologous promoter present in the baculovirus Autographa californica nucleopolyhedrovirus (AcMNPV) as described in Liang CY et al., 2004 (Arch Virol. 149: 51-60).

Recombinant viral vectors are useful for *in vivo* expression of the isolated antibody of the present disclosuresince they offer advantages such as lateral infection and targeting specificity. Lateral infection is inherent in the life cycle of, for example, retrovirus and is the process by which a single infected cell produces many progeny virions that bud off and infect neighboring cells. The result is that a large area becomes rapidly infected, most of which was not initially infected by the original viral particles. This is in contrast to vertical-type of infection in which the infectious agent spreads only through daughter progeny. Viral vectors can also be produced that are unable to spread laterally. This characteristic can be useful if the desired purpose is to introduce a specified gene into only a localized number of targeted cells.

Various methods can be used to introduce the expression vector of the present disclosureinto cells. Such methods are generally described in Sambrook et al., Molecular Cloning: A Laboratory Manual, Cold Springs Harbor Laboratory, New York (1989, 1992), in Ausubel et al., Current Protocols in Molecular Biology, John Wiley and Sons, Baltimore, Md. (1989), Chang et al., Somatic Gene Therapy, CRC Press, Ann Arbor, Mich. (1995), Vega et al., Gene Targeting, CRC Press, Ann Arbor Mich. (1995), Vectors: A Survey of Molecular Cloning Vectors and Their Uses, Butterworths, Boston Mass. (1988) and Gilboa et at. [Biotechniques 4 (6): 504-512, 1986] and include, for example, stable or transient transfection, lipofection, electroporation and infection with recombinant viral vectors. In addition, see U.S. Pat. Nos. 5,464,764 and 5,487,992 for positive-negative selection methods.

Introduction of nucleic acids by viral infection offers several advantages over other methods such as lipofection and electroporation, since higher transfection efficiency can be obtained due to the infectious nature of viruses.

Currently preferred *in vivo* nucleic acid transfer techniques (*in vivo* gene therapy) include transfection with viral or non-viral constructs, such as adenovirus, lentivirus, Herpes simplex I virus, or adeno-associated virus (AAV) and lipid-based systems. Useful lipids for lipid-mediated transfer of the gene are, for example, DOTMA, DOPE, and DC-Chol [Tonkinson et al., Cancer Investigation, 14(1): 54-65 (1996)]. The most preferred constructs for use in gene therapy are viruses, most preferably adenoviruses, AAV, lentiviruses, or retroviruses. A viral construct such as a retroviral construct includes at least one transcriptional promoter/enhancer or locus-defining element(s), or other elements that control gene expression by other means such as alternate splicing, nuclear RNA export, or post-translational modification of messenger. Such vector constructs also include a packaging signal, long terminal repeats (LTRs) or portions thereof, and positive and negative strand primer binding sites appropriate to the virus used, unless it is already present in the viral construct. In addition, such a construct typically includes a signal sequence for secretion of the peptide from a host cell in which it is placed. Preferably the signal sequence for this purpose is a mammalian signal sequence or the signal sequence of the polypeptide variants of the present disclosure. Optionally, the construct may also include a signal that directs polyadenylation, as well as one or more restriction sites and a translation termination sequence. By way of example, such constructs will typically include a 5' LTR, a tRNA binding site, a packaging signal, an origin of second-strand DNA synthesis, and a 3' LTR or a portion thereof. Other vectors can be used that are non-viral, such as cationic lipids, polylysine, and dendrimers.

Other than containing the necessary elements for the transcription and translation of the inserted coding sequence, the expression construct of the present disclosurecan also include sequences engineered to enhance stability, production, purification, yield or toxicity of the expressed peptide. For example, the expression of a fusion protein or a cleavable fusion protein comprising Met variant of the present disclosureand a heterologous protein can be engineered. Such a fusion protein can be designed so that the fusion protein can be readily isolated by affinity chromatography; e.g., by immobilization on a column specific for the heterologous protein. Where a cleavage site is engineered between the Met moiety and the heterologous protein, the Met moiety can be released from the chromatographic column by treatment with an appropriate enzyme or agent that disrupts the cleavage site [e.g., see Booth et al. (1988) Immunol. Lett. 19:65-70; and Gardella et al., (1990) J. Biol. Chem. 265:15854-15859].

It will be appreciated that a variety of prokaryotic or eukaryotic cells can be used as host-expression systems to express the isolated recombinant antibody (the polypeptide) of the present disclosure. These include, but are not limited to, microorganisms, such as bacteria transformed with a recombinant bacteriophage DNA, plasmid DNA or cosmid DNA expression vector containing the coding sequence; yeast transformed with recombinant yeast expression vectors containing the coding sequence; plant cell systems infected with recombinant virus expression vectors (e.g., cauliflower mosaic virus, CaMV; tobacco mosaic virus, TMV) or transformed with recombinant plasmid expression vectors, such as Ti plasmid, containing the coding sequence. Mammalian expression systems can also be used to express the polypeptides of the present disclosure.

Examples of bacterial constructs include the pET series of E. coli expression vectors [Studier et al. (1990) Methods in Enzymol. 185:60-89).

In yeast, a number of vectors containing constitutive or inducible promoters can be used, as disclosed in U.S. Pat. Application No: 5,932,447. Alternatively, vectors can be used which promote integration of foreign DNA sequences into the yeast chromosome.

In cases where plant expression vectors are used, the expression of the coding sequence can be driven by a number of promoters. For example, viral promoters such as the 35S RNA and 19S RNA promoters of CaMV [Brisson et al. (1984) Nature 310:511-514], or the coat protein promoter to TMV [Takamatsu et al. (1987) EMBO J. 6:307-311] can be used. Alternatively, plant promoters such as the small subunit of RUBISCO [Coruzzi et al. (1984) EMBO J. 3:1671-1680 and Brogli et al., (1984) Science 224:838-843] or heat shock promoters, e.g., soybean hsp17.5-E or hsp17.3-B [Gurley et al. (1986) Mol. Cell. Biol. 6:559-565] can be used. These constructs can be introduced into plant cells using Ti plasmid, Ri plasmid, plant viral vectors, direct DNA transformation, microinjection, electroporation and other techniques well known to the skilled artisan. See, for example, Weissbach & Weissbach, 1988, Methods for Plant Molecular Biology, Academic Press, NY, Section VIII, pp 421-463.

Other expression systems such as insects and mammalian host cell systems are well known in the art and can also be used by the present invention.

Recovery of the recombinant polypeptide is effected following an appropriate time in culture. The phrase "recovering the recombinant polypeptide" refers to collecting the whole fermentation medium containing the polypeptide and need not imply additional steps of separation or purification. Not withstanding the above, polypeptides of the present disclosurecan be purified using a variety of standard protein purification techniques, such as, but not limited to, affinity chromatography, ion exchange chromatography, filtration, electrophoresis, hydrophobic interaction chromatography, gel filtration chromatography, reverse phase chromatography, chromatofocusing and differential solubilization.

According to preferred embodiments of the present disclosure, the method according to this aspect of the present disclosurefurther comprising a step of detecting apoptosis in the cancer cells or the viral infected cells following the administration or the expression of the recombinant isolated antibody of the present disclosure.

The apoptosis induced by the recombinant isolated antibody of the present disclosurecan be detected using various methods known in the art.

***Ethidium homodimer-1 staining -*** Apoptosis can be detected by dyes specifically binding to cells with compromised membranes, *i.e*., dead cells. Briefly, unfixed cells such as cells in suspension, tissue culture, tissue sections and the like are stained with the fluorescent dye Ethidium homodimer-1 (excitation, 495 nm; emission, 635 nm). In this assay, live cells have a green fluorescent cytoplasm but no EthD-1 signal, whereas dead cells lack the green fluorescence and are stained with EthD-1.

***Tunnel assay*** (***Roche***, ***Basel, Switzerland) -*** labels DNA breaks which are characteristics of cells undergoing apoptosis with fluorescein (excitation 450-500 nm, emission 515-565 nm).

***Live*/*dead viability*/*cytotoxicity two-color fluorescence assay (Molecular Probes***, ***Inc., L-3224, Eugene, OR, USA) -*** This assay measures intracellular esterase activity with a cell-permeable substrate (Calcein-AM) which is converted by live cells to a fluorescent derivative (polyanion calcein, excitation, 495 nm; emission, 515 nm) which is thereafter retained by the intact plasma membrane of live cells.

***FACS analysis -*** using molecules capable of specifically binding cells undergoing apoptosis, such as propidium iodide and Annexin V (see the description of drawings and the Examples section which follows). Annexins V is a human protein characterized by calcium-mediated, high affinity binding to phosphatidylserine which undergoes externalization to the outer side of the plasma membrane during early apoptosis.

*D**NA fragmentation by gel electrophoresis -*** Briefly, DNA is extracted from unfixed cells and is subjected to gel electrophoresis (e.g., 1.5-2 % agarose gel) and the degree of DNA fragmentation is evaluated using any DNA stain such as Ethidium bromide, Syber Green and the like.

As is further shown in Figure 6 and is described in Example 3 of the Examples section which follows, the naked scFv G1 antibody was capable of inhibiting protein synthesis of the 526, 624 and 501A melanoma cells (and thus, "killing" the melanoma cells) in a peptide specific, MHC-restricted manner. In addition, inhibition of protein synthesis was dose-dependent with an IC₅₀ *(i.e.,* the concentration of the antibody required to achieve 50 % inhibition of protein synthesis) of 60 µg/ml for all HLA-A2+/gp100+ melanoma cells.

These results strongly suggest the use of the TCR-like recombinant isolated antibodies of the present disclosurein treating cancer or pathogen infection.

Thus, according to another aspect of the present disclosure, there is provided a method of treating cancer or pathogen infection. The method is effected by contacting to, or expressing in cells of a subject in need thereof a therapeutically effective amount of a recombinant isolated antibody capable of specifically binding an MHC-peptide complex, the recombinant isolated antibody being capable of inducing apoptosis of cancer cells or pathogen infected cells, the cancer cells or the pathogen infected cells specifically expressing the MHC-peptide complex, thereby treating the cancer or the pathogen infection in the subject.

The term "treating" refers to inhibiting or arresting the development of a disease, disorder or condition (e.g., cancer or pathogen infection) and/or causing the reduction, remission, or regression of a disease, disorder or condition. Those of skill in the art will understand that various methodologies and assays can be used to assess the development of a disease, disorder or condition, and similarly, various methodologies and assays may be used to assess the reduction, remission or regression of a disease, disorder or condition.

As used herein, the term "subject" (or "individual" which is interchangeably used herein) includes mammals, preferably human beings at any age which suffers from the disease, disorder or condition. Preferably, this term encompasses individuals who are at risk to develop the disease, disorder or condition. For example, individuals who express low levels of a cancer differentiation protein in a pre-malignant state, or individuals who are infected with a pathogen which is still in a latent phase.

As used herein the phrase "cells of a subject" includes any cells which are derived from the subject and are taken out of the subject (*i.e*., *ex vivo* gene therapy) or cells which are part of the subject (*i.e*., *in vivo* gene therapy as described hereinabove).

It will be appreciated that for *ex vivo* gene therapy, the cells which are derived from subject (either the subject in need of treatment or a donor subject) are cultured in the presence of the appropriate tissue culture medium and are transfected *ex vivo* with an expression vector containing the polynucleotide designed to express and secrete the recombinant isolated antibody of the present disclosureto the target cells (e.g., cancer cells or viral-infected cells of the subject), essentially as described hereinabove. Such cells can be for example, kidney cells, bone marrow cells, keratinocyte cells and lymphocyte cells.

Administration of the antibody-expressing cells of the present disclosurecan be effected using any suitable route such as intravenous, intra peritoneal, intra kidney, intra gastrointestinal track, subcutaneous, transcutaneous, intramuscular, intracutaneous, intrathecal, epidural and rectal. According to presently preferred embodiments, the antibody-expressing cells of the present disclosureare introduced to the individual using intravenous, intra kidney, intra gastrointestinal track and/or intra peritoneal administrations.

The antibody-expressing cells of the present disclosurecan be derived from either autologous sources such as self bone marrow cells or from allogeneic sources such as bone marrow or other cells derived from non-autologous sources. Since non-autologous cells are likely to induce an immune reaction when administered to the body several approaches have been developed to reduce the likelihood of rejection of non-autologous cells. These include either suppressing the recipient immune system or encapsulating the non-autologous cells or tissues in immunoisolating, semipermeable membranes before transplantation.

Encapsulation techniques are generally classified as microencapsulation, involving small spherical vehicles and macroencapsulation, involving larger flat-sheet and hollow-fiber membranes (Uludag, H. et al. Technology of mammalian cell encapsulation. Adv Drug Deliv Rev. 2000; 42: 29-64).

Methods of preparing microcapsules are known in the arts and include for example those disclosed by Lu MZ, et al., Cell encapsulation with alginate and alpha-phenoxycinnamylidene-acetylated poly(allylamine). Biotechnol Bioeng. 2000, 70: 479-83, Chang TM and Prakash S. Procedures for microencapsulation of enzymes, cells and genetically engineered microorganisms. Mol Biotechnol. 2001, 17: 249-60, and Lu MZ, et al., A novel cell encapsulation method using photosensitive poly(allylamine alpha-cyanocinnamylideneacetate). J Microencapsul. 2000, 17: 245-51.

For example, microcapsules are prepared by complexing modified collagen with a ter-polymer shell of 2-hydroxyethyl methylacrylate (HEMA), methacrylic acid (MAA) and methyl methacrylate (MMA), resulting in a capsule thickness of 2-5 µm. Such microcapsules can be further encapsulated with additional 2-5 µm ter-polymer shells in order to impart a negatively charged smooth surface and to minimize plasma protein absorption (Chia, S.M. et al. Multi-layered microcapsules for cell encapsulation Biomaterials. 2002 23: 849-56).

Other microcapsules are based on alginate, a marine polysaccharide (Sambanis, A. Encapsulated islets in diabetes treatment. Diabetes Thechnol. Ther. 2003, 5: 665-8) or its derivatives. For example, microcapsules can be prepared by the polyelectrolyte complexation between the polyanions sodium alginate and sodium cellulose sulphate with the polycation poly(methylene-co-guanidine) hydrochloride in the presence of calcium chloride.

It will be appreciated that cell encapsulation is improved when smaller capsules are used. Thus, the quality control, mechanical stability, diffusion properties, and *in vitro* activities of encapsulated cells improved when the capsule size was reduced from 1 mm to 400 µm (Canaple L. et al., Improving cell encapsulation through size control. J Biomater Sci Polym Ed. 2002;13: 783-96). Moreover, nanoporous biocapsules with well-controlled pore size as small as 7 nm, tailored surface chemistries and precise microarchitectures were found to successfully immunoisolate microenvironments for cells (Williams D. Small is beautiful: microparticle and nanoparticle technology in medical devices. Med Device Technol. 1999, 10: 6-9; Desai, T.A. Microfabrication technology for pancreatic cell encapsulation. Expert Opin Biol Ther. 2002, 2: 633-46).

The recombinant isolated antibody of the present disclosure, the nucleic acid construct encoding same or the cells expressing same can be administered to an organism per se, or in a pharmaceutical composition where it is mixed with suitable carriers or excipients.

As used herein a "pharmaceutical composition" refers to a preparation of one or more of the active ingredients described herein with other chemical components such as physiologically suitable carriers and excipients. The purpose of a pharmaceutical composition is to facilitate administration of a compound to an organism.

Herein the term "active ingredient" refers to the recombinant isolated antibody of the present disclosureor the polynucleotide or the cells expressing same accountable for the biological effect.

Hereinafter, the phrases "physiologically acceptable carrier" and "pharmaceutically acceptable carrier" which may be interchangeably used refer to a carrier or a diluent that does not cause significant irritation to an organism and does not abrogate the biological activity and properties of the administered compound. An adjuvant is included under these phrases.

Herein the term "excipient" refers to an inert substance added to a pharmaceutical composition to further facilitate administration of an active ingredient. Examples, without limitation, of excipients include calcium carbonate, calcium phosphate, various sugars and types of starch, cellulose derivatives, gelatin, vegetable oils and polyethylene glycols.

Techniques for formulation and administration of drugs may be found in "Remington's Pharmaceutical Sciences," Mack Publishing Co., Easton, PA, latest edition.

Suitable routes of administration may, for example, include oral, rectal, transmucosal, especially transnasal, intestinal or parenteral delivery, including intramuscular, subcutaneous and intramedullary injections as well as intrathecal, direct intraventricular, intracardiac, intravenous, inrtaperitoneal, intranasal, or intraocular injections.

Alternately, one may administer the pharmaceutical composition in a local rather than systemic manner, for example, via injection of the pharmaceutical composition directly into a tissue region of a patient.

Pharmaceutical compositions of the present disclosuremay be manufactured by processes well known in the art, e.g., by means of conventional mixing, dissolving, granulating, dragee-making, levigating, emulsifying, encapsulating, entrapping or lyophilizing processes.

Pharmaceutical compositions for use in accordance with the present disclosurethus may be formulated in conventional manner using one or more physiologically acceptable carriers comprising excipients and auxiliaries, which facilitate processing of the active ingredients into preparations which, can be used pharmaceutically. Proper formulation is dependent upon the route of administration chosen.

For injection, the active ingredients of the pharmaceutical composition may be formulated in aqueous solutions, preferably in physiologically compatible buffers such as Hank's solution, Ringer's solution, or physiological salt buffer. For transmucosal administration, penetrants appropriate to the barrier to be permeated are used in the formulation. Such penetrants are generally known in the art.

For oral administration, the pharmaceutical composition can be formulated readily by combining the active compounds with pharmaceutically acceptable carriers well known in the art. Such carriers enable the pharmaceutical composition to be formulated as tablets, pills, dragees, capsules, liquids, gels, syrups, slurries, suspensions, and the like, for oral ingestion by a patient. Pharmacological preparations for oral use can be made using a solid excipient, optionally grinding the resulting mixture, and processing the mixture of granules, after adding suitable auxiliaries if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethylcellulose, sodium carbomethylcellulose; and/or physiologically acceptable polymers such as polyvinylpyrrolidone (PVP). If desired, disintegrating agents may be added, such as cross-linked polyvinyl pyrrolidone, agar, or alginic acid or a salt thereof such as sodium alginate.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions and suitable organic solvents or solvent mixtures. Dyestuffs or pigments may be added to the tablets or dragee coatings for identification or to characterize different combinations of active compound doses.

Pharmaceutical compositions which can be used orally, include push-fit capsules made of gelatin as well as soft, sealed capsules made of gelatin and a plasticizer, such as glycerol or sorbitol. The push-fit capsules may contain the active ingredients in admixture with filler such as lactose, binders such as starches, lubricants such as talc or magnesium stearate and, optionally, stabilizers. In soft capsules, the active ingredients may be dissolved or suspended in suitable liquids, such as fatty oils, liquid paraffin, or liquid polyethylene glycols. In addition, stabilizers may be added. All formulations for oral administration should be in dosages suitable for the chosen route of administration.

For buccal administration, the compositions may take the form of tablets or lozenges formulated in conventional manner.

For administration by nasal inhalation, the active ingredients for use according to the present disclosureare conveniently delivered in the form of an aerosol spray presentation from a pressurized pack or a nebulizer with the use of a suitable propellant, e.g., dichlorodifluoromethane, trichlorofluoromethane, dichlorotetrafluoroethane or carbon dioxide. In the case of a pressurized aerosol, the dosage unit may be determined by providing a valve to deliver a metered amount. Capsules and cartridges of, e.g., gelatin for use in a dispenser may be formulated containing a powder mix of the compound and a suitable powder base such as lactose or starch.

The pharmaceutical composition described herein may be formulated for parenteral administration, e.g., by bolus injection or continuous infusion. Formulations for injection may be presented in unit dosage form, e.g., in ampoules or in multidose containers with optionally, an added preservative. The compositions may be suspensions, solutions or emulsions in oily or aqueous vehicles, and may contain formulatory agents such as suspending, stabilizing and/or dispersing agents.

Pharmaceutical compositions for parenteral administration include aqueous solutions of the active preparation in water-soluble form. Additionally, suspensions of the active ingredients may be prepared as appropriate oily or water based injection suspensions. Suitable lipophilic solvents or vehicles include fatty oils such as sesame oil, or synthetic fatty acids esters such as ethyl oleate, triglycerides or liposomes. Aqueous injection suspensions may contain substances, which increase the viscosity of the suspension, such as sodium carboxymethyl cellulose, sorbitol or dextran. Optionally, the suspension may also contain suitable stabilizers or agents which increase the solubility of the active ingredients to allow for the preparation of highly concentrated solutions.

Alternatively, the active ingredient may be in powder form for constitution with a suitable vehicle, e.g., sterile, pyrogen-free water based solution, before use.

The pharmaceutical composition of the present disclosuremay also be formulated in rectal compositions such as suppositories or retention enemas, using, e.g., conventional suppository bases such as cocoa butter or other glycerides.

Pharmaceutical compositions suitable for use in context of the present disclosureinclude compositions wherein the active ingredients are contained in an amount effective to achieve the intended purpose. More specifically, a therapeutically effective amount means an amount of active ingredients (the recombinant isolated antibody of the present disclosureor the polynucleotide or the cells expressing same) effective to prevent, alleviate or ameliorate symptoms of a disorder (e.g., cancer or viral infection) or prolong the survival of the subject being treated.

Determination of a therapeutically effective amount is well within the capability of those skilled in the art, especially in light of the detailed disclosure provided herein.

For any preparation used in the methods of the disclosure, the therapeutically effective amount or dose can be estimated initially from *in vitro* and cell culture assays. For example, a dose can be formulated in animal models to achieve a desired concentration or titer. Such information can be used to more accurately determine useful doses in humans.

Toxicity and therapeutic efficacy of the active ingredients described herein can be determined by standard pharmaceutical procedures *in vitro*, in cell cultures or experimental animals. The data obtained from these *in vitro* and cell culture assays and animal studies can be used in formulating a range of dosage for use in human. The dosage may vary depending upon the dosage form employed and the route of administration utilized. The exact formulation, route of administration and dosage can be chosen by the individual physician in view of the patient's condition. (See e.g., Fingl, et al., 1975, in "The Pharmacological Basis of Therapeutics", Ch. 1 p.1).

For example, as is shown in Figure 6 and in the Examples section which follows, the G1 scFV antibody caused a 50 % inhibition of protein synthesis in melanoma cells at a concentration of 60 µg/ml, and an 80 % inhibition at a concentration of 120 µg/ml. In addition, as is shown in Table 2 of the Examples section which follows, while the 2F1 ScFv antibody exhibited an IC₅₀ of 130 µg/ml on protein synthesis in melanoma cells, the whole IgG clone which is derived from such Fab fragment exhibited a significantly lower IC₅₀ of 4 µg/ml.

In addition, as is shown in Figures 7a-c, 8d-e and is described in the Examples section which follows, when immunotoxins were utilized inhibition of melanoma cells was achieved with IC₅₀ concentrations of 10 ng/ml of the G1 scFv PE38 (Figure 7a), 15 ng/ml of the 2F1 scFv PE38 (Figure 8d) and 10-100 ng/ml of the CLA12 scFv PE38 (Figure 8e).

Dosage amount and interval may be adjusted individually to provide the cancer cells or viral cells with levels of the active ingredient which are sufficient to induce apoptosis (minimal effective concentration, MEC). The MEC will vary for each preparation, but can be estimated from in vitro data. Dosages necessary to achieve the MEC will depend on individual characteristics and route of administration. Detection assays can be used to determine plasma concentrations.

Depending on the severity and responsiveness of the condition to be treated, dosing can be of a single or a plurality of administrations, with course of treatment lasting from several days to several weeks or until cure is effected or diminution of the disease state is achieved.

The amount of a composition to be administered will, of course, be dependent on the subject being treated, the severity of the affliction, the manner of administration, the judgment of the prescribing physician, etc.

Compositions of the present disclosure may, if desired, be presented in a pack or dispenser device, such as an FDA approved kit, which may contain one or more unit dosage forms containing the active ingredient. The pack may, for example, comprise metal or plastic foil, such as a blister pack. The pack or dispenser device may be accompanied by instructions for administration. The pack or dispenser may also be accommodated by a notice associated with the container in a form prescribed by a governmental agency regulating the manufacture, use or sale of pharmaceuticals, which notice is reflective of approval by the agency of the form of the compositions for human or veterinary administration. Such notice, for example, may be of labeling approved by the U.S. Food and Drug Administration for prescription drugs or of an approved product insert. Compositions comprising a preparation of the disclosure formulated in a compatible pharmaceutical carrier may also be prepared, placed in an appropriate container, and labeled for treatment of an indicated condition, as is further detailed above.

It will be appreciated that the method of the present disclosurecan be also used to treat various other pathologies which are associated with reduced levels of apoptosis. These include, for example, psoriasis (Victor FC and Gottlieb AB, 2002, J. Drugs Dermatol. 1: 264-75), ichthyosis (Melino G, et al., 2000, Methods Enzymol. 322: 433-72), common warts, keratoacanthoma (Tsuji T, 1997, J. Cutan. Pathol. 24: 409-15), seborrhoic keratosis (Satchell AC, et al., 2004, Br. J. Dermatol. 151: 42-9), seborrhea, squamous cell carcinomas (SCC; Seta C, et al., 2000, J. Oral Pathol. Med. 29: 271-8), basal cell carcinoma (BCC; Li C, et al., 2004, Oncogene. 2004, 23: 1608-17), non-melanoma skin cancer (NMSC) and multiple human tumors.

While further reducing the present invention to practice, the present inventors have uncovered that the recombinant isolated antibody of the present disclosurecan be used to diagnose cancer or pathogen infection. As is shown in Figure 4a-f and is described in Example 2 of the Examples section which follows, the G1 scFv antibody was capable of specifically immunostaining the HLA-A2-positive/gp100 expressing FM-3-29, M-ww, M-PAT, M-141 and FM3-D natural melanoma cells, but not the HLA-A2 negative/gp100 expressing G-43 natural melanoma cells.

Thus, according to an additional aspect of the present disclosure there is provided a method of diagnosing cancer or viral infection in a subject. The method is effected by (a) contacting a biological sample of the subject with a recombinant isolated antibody capable of specifically binding an MHC-peptide complex under conditions suitable for immunocomplex formation; the recombinant isolated antibody being capable of inducing apoptosis in target cancer cells or viral infected cells, and (b) detecting formation of the immunocomplex, thereby diagnosing the cancer or viral infection in the subject.

As used herein the phrase "diagnosing" refers to classifying a disease (cancer or pathogen infection) or a symptom, determining a severity of the disease, monitoring disease progression, forecasting an outcome of a disease and/or prospects of recovery.

As used herein "biological sample" refers to a sample of tissue or fluid isolated from a subject, including but not limited to, for example, blood cells, bone marrow cells and specifically macrophages, lymph fluid, various tumors, neuronal cells, dendritic cells, organs, and also samples of *in vivo* cell culture constituents. It should be noted that a "biological sample of the subject" may also optionallycomprise a sample that has not been physically removed from the subject.

According to one preferred embodiments of the present disclosure, the cancer which is diagnosed using the method according to this aspect of the present disclosureis a solid tumor (e.g., breast cancer, melanoma and the like). Preferably, the biological sample obtained from the subject is a tissue specimen such as a tissue biopsy which is preferably cut into sections (e.g., paraffin sections or cryosections).

Diagnosis of cancer or pathogen infection according to the present disclosurecan be effected by contacting the biological sample of the subject with the recombinant isolated antibody of the present disclosureunder conditions suitable for immunocomplex formation.

As used herein the term "immunocomplex" refers to a complex formed between an antibody (e.g., the recombinant isolated antibody of the present disclosureas described hereinabove) and its specific antigen (e.g., a specific MHC-peptide complex such as the HLA-A2-G9-209 complex, the HLA-A2-G9-280 complex, the HLA-A2-MART126-35 complex).

The immunocomplex of the present disclosurecan be formed at a variety of temperatures, salt concentration and pH values which may vary depending on the antibody used and the cells presenting the antigen and those of skills in the art are capable of adjusting the conditions suitable for the formation of each immunocomplex.

For example, as described in Example 2 and in the description of Figures 4a-g, for the formation of the G1-HLA-A2-G9-209 immunocomplex, melanoma cells (e.g., FM-3-29, M-ww, M-PAT, M-141 cells) were incubated for 1 hour on ice with 20-30 µg G1 scFv in RPMI culture medium containing 10 % fetal calf serum (FCS).

According to the method of this aspect of the present disclosure, detection of immunocomplex formation is indicative of a diagnosis of the disease, e.g., cancer or viral infection. Various methods can be used to detect the immunocomplex of the present disclosure and those of skills in the art are capable of determining which method is suitable for each immunocomplex and/or the type of cells used for diagnosis.

For example, the immunocomplex can be detected by conventional immunohistochemistry or immunofluorescence, FACS, ELISA, Western blot and RIA analyses, or by a molecular weight-based approach.

***Immunohistochemistry or immunofluorescence analyses -*** This method involves detection of an antigen (e.g., the MHC-peptide complex) *in situ* in fixed cells by antigen specific antibodies (*i.e*., the recombinant isolated antibody of the present disclosure). The antigen specific antibodies may be enzyme linked or linked to fluorophores. Detection is by microscopy and subjective or automatic evaluation. If enzyme linked antibodies are employed, a colorimetric reaction may be required. It will be appreciated that immunohistochemistry is often followed by counterstaining of the cell nuclei using for example Hematoxyline or Giemsa stain.

***Fluorescence activated cell sorting (FACS) -*** This method involves detection of an antigen *in situ* in cells by antigen specific antibodies. The antigen specific antibodies are linked to fluorophores. Detection is by means of a cell sorting machine which reads the wavelength of light emitted from each cell as it passes through a light beam. This method may employ two or more antibodies simultaneously.

***Enzyme linked immunosorbent assay (ELISA) -*** This method involves fixation of a sample (e.g., fixed cells or a proteinaceous solution) containing an antigen (e.g., the MHC-peptide complex) to a surface such as a well of a microtiter plate. An antigen specific antibody coupled to an enzyme is applied and allowed to bind to the antigen. Presence of the antibody is then detected and quantitated by a colorimetric reaction employing the enzyme coupled to the antibody. Enzymes commonly employed in this method include horseradish peroxidase and alkaline phosphatase. If well calibrated and within the linear range of response, the amount of substrate present in the sample is proportional to the amount of color produced. A substrate standard is generally employed to improve quantitative accuracy.

***Western blot** -* This method involves separation of a substrate from other protein by means of an acrylamide gel followed by transfer of the substrate to a membrane (e.g., nylon or PVDF). Presence of the substrate is then detected by antibodies specific to the substrate, which are in turn detected by antibody binding reagents. Antibody binding reagents may be, for example, protein A, or other antibodies such as those in the ECL kit (Amersham Biosciences Inc, Piscataway, NJ, USA). Antibody binding reagents may be radiolabeled or enzyme linked as described hereinabove. Detection may be by autoradiography, colorimetric reaction or chemiluminescence. This method allows both quantitation of an amount of substrate and determination of its identity by a relative position on the membrane which is indicative of a migration distance in the acrylamide gel during electrophoresis. It will be appreciated that in the case of the MHC-peptide complex, a non-denaturing gel electrophoresis is preferably employed.

*Radio-immunoassay **(RIA):*** In one version, this method involves precipitation of the desired antigen (*i.e*., the MHC-peptide complex) with a specific antibody and radiolabeled antibody binding protein (e.g., protein A labeled with I¹²⁵) immobilized on a precipitable carrier such as agarose beads. The number of counts in the precipitated pellet is proportional to the amount of antigen.

In an alternate version of the RIA, a labeled antigen and an unlabelled antibody binding protein are employed. A sample containing an unknown amount of antigen is added in varying amounts. The decrease in precipitated counts from the labeled antigen is proportional to the amount of antigen in the added sample.

***Molecular weight***-***based approach*** - It will be appreciated that the immunocomplex formed between the MHC-peptide complex exhibits a higher molecular weight than its components, i.e., the recombinant isolated antibody or the MHC-peptide complex. Thus, methods capable of detecting such a change in the molecular weight can be also employed. For example, the immunocomplex can be detected by a gel retardation assay. Briefly, a non-denaturing acrylamide gel is loaded with samples containing the recombinant isolated antibody and the MHC-peptide complex before and after immunocomplex formation. A shift in the size (molecular weight) of the protein product as compared with its components is indicative of the presence of an immunocomplex. Such a shift to a higher molecular weight can be viewed using a non-specific protein staining such as silver stain or commassie blue stain. Alternatively, the MHC or recombinant isolated antibody can be labeled (e.g., with a radioactive label) prior to gel electrophoresis. Additionally or alternatively, cells expressing the MHC complex can be radioactively labeled prior to protein extraction.

Preferably, the method of detecting the formation of the immunocomplex is immunohistochemistry (including immunofluorescence) which is performed on tissue sections (e.g., paraffin sections or cryosections) of a solid tumor.

It will be appreciated that diagnosing of the cancer or pathogen infection can be also effected by detecting apoptosis (as described hereinabove) in the cells following contacting the cells with the recombinant isolated antibody of the present disclosure.

Therefore, according to yet an additional aspect of the present disclosure there is provided a method of diagnosing cancer or pathogen infection in a subject. The method is effected by (a) contacting a biological sample of the subject with a recombinant isolated antibody capable of specifically binding an MHC-peptide complex under conditions suitable for immunocomplex formation, the recombinant isolated antibody being capable of inducing apoptosis in target cancer cells or pathogen infected cells; and (b) detecting a level of apoptosis in cells of the biological sample thereby diagnosing the cancer or pathogen infection in the subject.

The agents described hereinabove for detection of immunocomplex formation may be included in a diagnostic kit/article of manufacture preferably along with appropriate instructions for use and labels indicating FDA approval for use in diagnosing and/or assessing a severity of cancer or viral infection.

Such a kit can include, for example, at least one container including at least one of the above described diagnostic agents (e.g., the G1, 9H or CLA12 antibodies) and an imaging reagent packed in another container (e.g., enzymes, secondary antibodies, buffers, chromogenic substrates, fluorogenic material). The kit may also include appropriate buffers and preservatives for improving the shelf-life of the kit.

According to preferred embodiments of the present disclosure, diagnosis of the cancer or viral infection can be also effected by detecting the level of apoptosis in the cells following contacting the recombinant isolated antibody with the cells. Such methods are described hereinabove.

As used herein the term "about" refers to ± 10 %.

Additional objects, advantages, and novel features of the present disclosurewill become apparent to one ordinarily skilled in the art upon examination of the following examples, which are not intended to be limiting. Additionally, each of the various embodiments and aspects of the present disclosureas delineated hereinabove and as claimed in the claims section below finds experimental support in the following examples.

### EXAMPLES

Reference is now made to the following examples, which together with the above descriptions, illustrate the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., Ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (Eds.) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., Ed. (1994); "Culture of Animal Cells - A Manual of Basic Technique" by Freshney, Wiley-Liss, N. Y. (1994), Third Edition; "Current Protocols in Immunology" Volumes I-III Coligan J. E., Ed. (1994); Stites et al. (Eds.), "Basic and Clinical Immunology" (8th Edition), Appleton & Lange, Norwalk, CT (1994); Mishell and Shiigi (Eds.), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., Ed. (1984); "Nucleic Acid Hybridization" Hames, B. D., and Higgins S. J., Eds. (1985); "Transcription and Translation" Hames, B. D., and Higgins S. J., Eds. (1984); "Animal Cell Culture" Freshney, R. I., Ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); "The function of MHC molecules" In "Fundamental Immunology", Paul WE (Ed.), 1999; Forth edition; Lippincott-Raven publishers;. Other general references are provided throughout this document. The procedures therein are believed to be well known in the art and are provided for the convenience of the reader.

### EXAMPLE 1

### PREPARATION OF TCR-LIKE ANTIBODIES

***Isolation and Characterization of TCR-like antibodies -*** The model system used in the present study was the human superhaplotype HLA-A2 molecule, which is the most frequent MHC allele in the Caucasian population (approximately 40 %). Recombinant MHC-peptide complexes displaying peptide T cell epitopes of peptides derived from tumor associated and viral antigens, were generated by using a single-chain MHC (scMHC) construct [Denkberg et. al., 2000; Denkberg et. al., 2001].

Lev et al., 2002, utilized recombinant-engineered single-chain MHC-peptide complexes to isolate antibodies with TCR-like specificity from a large non-immune repertoire of phage antibody library. The target HLA-A2/peptide complexes screened included a variety of epitopes derived from tumor associated antigens such as the telomerase catalytic subunit (hTERT) widely expressed in many tumor cells, the melanoma differentiation antigen gp100 [Denkberg et al., 2002a], the epithelial cell-associated mucin (MUC1) related to breast carcinomas [Cohen et al., 2002] or viral epitopes derived from human T cell lymphotropic virus type I (HTLV-1) transcription factor (TAX) [Cohen et al 2003], influenza matrix protein epitope or Epstein-Bar virus (EBV)-derived epitope.

After two and three cycles of selection on peptide-MHC complexes, an unexpected and surprising high frequency (6-80 %) of antibodies recognizing human HLA-A2 MHC molecules complexed with various peptides was observed. Moreover, a significantly high proportion of these antibodies (20-80 %) exhibited MHC-restricted, peptide specific binding properties like T cells. Unlike previous reports in murine systems where only a single rare antibody clone could be isolated from hybridoma or a phage display library, here a panel of different antibodies for each MHC-peptide complex screened was isolated [Cohen et al., 2002; Lev et al., 2002; Denkberg et al., 2002a; Cohen et al 2003]. These antibodies, with antigen-specific, MHC-restricted specificity of T cells, bound to their corresponding MHC-peptide complex with a high affinity in the nanomolar range. In addition, these antibodies were selective to the MHC complex only when the specific peptide was displayed. Hence, these antibodies exhibit binding properties and kinetics of antibodies but mimic the fine specificity of T cells and were thus termed TCR-like antibodies.

To demonstrate the specific binding of the isolated antibody fragments (scFv) to MHC-peptide complex in the native form, as expressed on the cell surface, several strategies were employed. These experiments demonstrated that specific scFv bound only to cells displaying the appropriate specific peptide (Denkberg, G., et al., 2002a; Lev, A., et al., 2002; Cohen, CJ., et al., 2002; Lev, A., et al., 2002, Human Recombinant Antibodies with MHC-restricted T Cell Receptor-Like Specificity: Recognition of distinct T cell epitopes derived from a variety of tumor associated antigens. Tumor Microenvironment: Progression, Therapy and Prevention Monduzzi Editore press Pp 163-167; Cohen, CJ., et al., 2003, J. Immunol 170: 4349-4361; Denkberg, G. et al., 2003, J. Immunol. 171:2197-2207; Cohen C.J., et al., 2003, J. Mol. Recog. 16: 324-332; Yamano, Y., et al., 2004, J. Exp. Med. 199: 1367-1377; Held, G., et al., 2004, Eur. J. Immunol. 34: 2919-2929). For example, ScFvs 1A7, 2F1 and G2D12 reacted only with their respective gp100 derived epitope G9-209, G9-280 and G9-154 loaded RMA-S-HHD cells and not with cells loaded with control peptides (Figures 1a-c). Several other APCs were used to demonstrate the peptide-specific binding of these Fabs, such as the TAP+ EBV-transformed B-lymphoblast HLA-A2+ JY cells or mature dendritic cells (DCs). In these cells, peptide loading is facilitated by the exchange of endogenously derived peptides with HLA-A2-restricted peptides supplied externally by incubation of the cells with the desired peptides. The TCR-like Fab fragments reacted with JY cells or mature DCs incubated with the specific peptide to which they were selected but not with control cells displaying other control HLA-A2-restricted peptides as shown in Figure 1b.

The unexpected high frequency of these antibodies and the ability to isolate several different antibodies directed to either complex even more surprising in view of previous reports, in which the use of immunized or naive phage libraries resulted in only a single antibody clone [Andersen et. al., 1996; Porgador et. al., 1997; Dadaglio et. al., 1997; Zhong et. al., 1997b; Krogsgaard et. al., 2000; Polakova et. al., 2000; Cohen et al., 2003].

***Study of antigen presentation using TCR-like antibodies -*** TCR-like antibodies are powerful tools for studying antigen presentation in transfected models as well as in tumor cells. For example, to examine the ability of the TCR-like Fab antibodies to detect HLA-A2/Tax complexes produced under physiological antigen processing conditions, the EBV-transformed B-cell HLA-A2-positive antigen presenting JY cells were transfected with the HTLV-1 Tax gene. Twenty-four hours after transfection, significant staining above control could be clearly seen using TCR-like antibodies only with HLA-A2-positive JY cells transfected with the Tax gene but not with HLA-A2-negative APD cells transfected with the Tax gene (Figures 2d-i). The peptide-specific, MHC-restricted pattern of reactivity by anti TAX/HLA-A2 Fabs was not due to differences in transfection efficiency or HLA expression of JY and APD cells because the percentage of transfected cells was similar as determined in control experiments utilizing a GFP construct. These results indicate that the TCR-like Fab antibodies are capable of detecting the specific MHC-peptide complex after active and naturally occurring endogenous intracellular processing.

Since the density of a particular peptide-HLA complex on tumor cells is expected to be low compared to peptide-pulsed or transfected APCs, the avidity of several scFvs was increased by making scFv tetramers, with directly tagged fluorescent probes. To form scFv tetramers, each scFv fragment was engineered to include a BirA sequence tag for site-specific biotinylation at the C-terminus of the CL or CHl domain, and following biotinylation, each streptavidin molecule was capable of binding four scFv fragments. Another advantage of using fluorescent tetramers is that only a single staining step is required, whereas monomeric unlabeled scFvs require a fluorescently labeled secondary antibody. Using these highly sensitive scFv-tetramers, it was possible to specifically detect the occurrence of desired peptide/MHC complexes on the surface of breast carcinoma cells. A tetramerized scFv directed towards a mucin epitope in complex with the HLA-A2 molecule was able to detect MUC1-D6 complexes on the surface of MUC1-expressing, HLA-A2+, MCF7 and MDA-MB 231 cells but not on MUC1-negative, HLA-A2+ melanoma FM3D cells nor on HLA-A2 and MUC1-negative G-43 cells. The M3A1 tetramer exhibited a low background staining with the MUC1-positive, HLA-A2-negative SK-BR3 cells. Such molecules provide also new means to quantitate specific peptide/MHC complexes as presented on the surface of APCs and tumor cells. This approach represents the first attempt to quantify, using a direct mean, the number of tumor-associated TCR ligands (peptide-MHC complexes) on the surface of tumor cells.

The fluorescence intensity of stained cells was measured and compared with that of calibration beads with known numbers of fluorophores (Cohen, CJ., et al., 2002, Cancer Res. 62: 5835-5844). This method provides an easy and sensitive means of quantifying fluorophore-stained cells with a flow cytometer. Anti mucin TCR-like Fab was used to initially quantitate the number of MUC1-D6/HLA-A2 complexes that are displayed on peptide-pulsed cells as well as naturally un-pulsed tumor cells: pulsing of JY APCs with peptide resulted in the ability to detect as many as 1.2 x 10⁵ D6-HLA-A2 complexes per cell. The latter result is in complete agreement with recent quantitation of murine H-2kb bound to the ovalbumin peptide SIINFEKL after recombinant vaccinia virus infection of cells *in vitro* using a murine specific TCR-like antibody [Porgador et al., 1997]. The number of D6-MUC1-derived complexes on the surface of MUC1 expressing tumor cells was estimated to reach only several hundreds per cell (Table 1, hereinbelow).

**Table 1**

| ***Quantitation of the number of D6-HLA-A2 complexes on the surface of tumor cells*** | |
|---|---|
| Cells | Mean number of sites/cell |
| JY pulsed (1 mM peptide) | 120,959 ± 16,934 |
| MCF7 pulsed (1 mM peptide) | 5,539 ± 1,828 |
| MDA MB 231 (1 mM peptide) | 10,022 ± 2,004 |
| MCF7 | 284 ± 170 |
| MDA MB 231 | 173 ± 112 |
| Background | 26 ± 11 |

Table 1: The fluorescence intensity of stained cells in each experiment was compared with fluorescence intensities of calibration beads with known numbers of phycoerthrin (PE) molecules per bead (QuantiBRITE PE beads, Becton-Dickinson) and the number of sites for each experiment was determined. The number of non-specific sites was determined by the intensity of staining of cells that do not express peptide or HLA-A2. The number of specific sites for each experiment was then calculated for each experiment. The mean number of sites was calculated from 10 independent experiments/determinations. The deviation in number of sites depend on the sensitivity of detection and the physiological status of the cells in each individual determination. Background number of sites was determined as described, using SK-BR3 (HLA-A2- /MUC1+), FM3D (HLA-A2+/MUC1-), and G-43 (HLA-A2-/MUC1-) cells as controls. JY cells are B cells. Pulsing with peptide serves as a mean to quantify potential total HLA-A2 molecules on the cell surface.

In a viral model, it was possible to detect HLA-A2/Tax complexes on HTLV-1 infected cells. HTLV-1 is involved in many pathologies including Adult T-cell lymphoma/leukemia (ATLL) and neurological disorders (HAM/TSP). A significant staining with anti-Tax/HLA-A2 Fab was observed on HTLV-1 infected RSCD4 (HLA-A2+) but not on HUT 102 cells (HLA-A2-), indicating that the TCR-like Fab is capable of detecting the specific HLA-A2/Tax complex on the surface of virus-infected cells. The staining pattern revealed two sub-populations with a high and low-moderate reactivity with the TCR-like Fab, which may indicate variability in the expression of the specific Tax epitope within subpopulations of RSCD4 HTLV-1-infected cells. Similar variability observed in staining experiments with an anti-Tax protein antibody. This analysis revealed that virus-infected RSCD4 cells, which could also be divided into two sub-populations with high and moderate reactivity, display on their surface 3 x 10⁴ HLA-A2/Tax complexes and several hundred sites respectively. These results clearly demonstrate the power of such TCR-like antibodies to provide useful and easily obtained direct quantitative data on the expression of specific peptide-MHC complexes on each cell in a population. In addition, these results demonstrate the high level expression of specific MHC class I molecules which display determinants derived from proteins produced upon transfection with an intact whole gene or directly on virus-infected cells.

These results are surprising in view of the fact that the number of MHC-peptide complexes on the surface of tumor cells is low and were measured to be in the range of few hundred complexes per cell (Table 1).

### EXAMPLE 2

### TCR-LIKE ANTIBODIES ARE CAPABLE OF RECOGNIZING SPECIFIC MHC-PEPTIDE COMPLEXES ON MELANOMA CELLS

To test the biological activity of a TCR-like antibody on tumor cells the antibody G1 which recognizes the HLA-A2 in a complex with the gp100-derived peptide 209, was employed in vitro, as follows. The procedures used in the experiments reported in Examples 2-5 (e.g., apoptosis, cell death, FACS, flow cytometry and antibody binding) are included in Denkberg, G., et al., 2002, Proc. Natl. Acad. Sci. USA. 99: 9421-9426; Lev, A., et al., 2002, Cancer Res. 62: 3184-3194; Denkberg, G., et al., 2003, J. Immunol. 171:2197-2207).

Melanoma tumor cells were used to determine the reactivity of the recombinant Fab or scFv antibodies with cell surface-expressed HLA-A2/peptide complexes. About 10⁶ cells were washed twice with serum-free RPMI and incubated for 60-90 minutes at 4 °C with recombinant TCR-like antibodies (10-100 µg/ml) in 100 µl. After three washes the cells were incubated with FITC-labeled anti-human Fab (Jackson Immunoresearch, West Grove, PA, USA). After a final wash, the cells were resuspended in ice-cold PBS. Adherent tumor cells were harvested by trypsinization and resuspended in cold RPMI. All subsequent washes and incubations were performed in ice-cold PBS. Analysis of the cells was performed by a FACStar flow cytometer (Becton Dickinson and Co, San Jose, CA, USA) and the results were analyzed with the WinMDI program (Trotter J., http://facs.scripps.edu/).

***Stable HLA-A2 complexes are formed on peptide-loaded cells -* To** demonstrate the ability of the G1 antibody to specifically bind an HLA-G9-209 complex, cells expressing the HLA-G9-209 or the HLA-G9-280 complexes, or unloaded cells were incubated with the W6/32 (an antibody directed against HLA), the BB7.2 (an antibody directed against the HLA-A2) or the G1 antibody. As is shown in Figures 3a-c, the expression and stabilization of the HLA molecules on the cell surface is dependent on an MHC-peptide complex. In addition, the G1 antibody was found to specifically interact with cells which present the MHC-G9-209 complex. To demonstrate that the G1 scFv can bind the specific MHC-peptide complex displayed on the surface of tumor cells the HLA-A2+ melanoma FM3D cells were used. The cells were pulsed with the specific gp100-derived G9-209 peptide or control peptide (Figure 3d). Similarly to the JY APCs, pulsing melanoma cells enables the display of the exogenously supplied peptide facilitated by peptide exchange. Using this strategy, a mixture of exogenously and endogenously derived peptides presented on HLA-A2 that are displayed on the cell surface was obtained. As shown in Figure 3e, the G1 scFv antibody was able to stain and detect HLA-A2/G9-209M complexes on G9-209M-pulsed FM3D cells but not on FM3D cells pulsed with the gp100-derived G9-280 peptide. The G1 scFv antibody was able also to stain FM3D cells pulsed with the native non-modified G9-209 peptide (Figure 3f), however control HLA-A2 negative KB3-1 cells (Figure 3g) pulsed with the G9-209M or G9-209 peptides were not recognized by the G1 scFv antibody (Figure 3h).

Altogether, these results demonstrate that the G1 scFv antibody can recognize the specific MHC-peptide complex on the surface of peptide-pulsed as well as non-pulsed tumor melanoma cells.

### EXAMPLE 3

### IDENTIFICATION OF THE 9H AND CLA12 RECOMBINANT ANTIBODIES

A large human synthetic single-chain Fv antibody library was screened for antibodies capable of binding MHC-peptide complexes. In this library the *in vivo* formed complementarity determining regions (CDRs) were shuffled combinatorially onto germline-derived human variable-region frameworks [Azriel-Rosenfeld R, Valensi M, Benhar I. A human synthetic combinatorial library of arrayable single-chain antibodies based on shuffling in vivo formed CDRs into general framework regions. J Mol Biol. 2004 Jan 2; 335(1):177-92].

Screening was performed essentially as described elsewhere (Denkberg, G., et al., 2002, Proc. Natl. Acad. Sci. USA. 99: 9421-9426) using the following antigens: the single chain HLA-A2-β2m molecule complexes with the G9-209 peptide derived from the melanoma gp100 protein and the single chain HLA-A2-β2m molecule complexed with the 26-35 peptide derived from the melanoma MART1 protein.

Two scFv antibody clones were isolated as soluble antibodies:
1. The 9H scFv antibody (scFv coding sequence is set forth by SEQ ID NO:3; scFv polypeptide sequence is set forth by SEQ ID NO:1; and 9H CDRs are provided in SEQ ID NOs:5-10) which is a human antibody specific to the HLA-A2-G9-209 complex with a binding affinity of 0.5 nM (not shown).
2. The CLA12 scFv antibody (scFv coding sequence is set forth by SEQ ID NO:18; scFv polypeptide sequence is set forth by SEQ ID NO:17; and CLA12 CDRs are provided in SEQ ID NOs:19-24) which is a human antibody specific to the HLA-A2-MART₂₆₋₃₅ peptide derived from the MART1 melanoma protein.

### EXAMPLE 4

### TCR-LIKE ANTIBODIES ARE CAPABLE OF DETECTING TUMOR-DERIVED MHC-COMPLEXES IN SITU

Another major potential use for TCR-like antibodies is the direct *in situ* visualization of ligand-bearing cells within undisturbed tissues using immunohistological methods. As a first step to assess this potential, the potential of G1 scFv to directly visualize ligand-bearing cells (HLA-A2/G9-209 complexes) was tested on melanoma cells.

Melanoma cells were derived from melanoma patients and were kindly provided from The National Cancer Institute, NIH.

***In** situ* ***detection of HLA-A2*/*G9-209 peptide-MHC complexes on melanoma cells -*** To further characterize the capacity of the G1 antibody to recognize the gp100-derived G9-209 epitope on cell surface, non-pulsed melanoma cells were employed in an immunohistochemistry analysis (Figures 4a-g). Melanoma gp100 expressing, HLA-A2-positive and negative melanoma cells were subjected to immuno-histochemistry staining with the G1 scFv antibody followed by a secondary staining step with HRP-labeled anti-Myc antibody. As shown in Figures 4a-g, these experiments showed a strong positive staining of the gp100 expressing, HLA-A2-positive melanoma lines FM-3-29 (Figure 4a), M-ww (Figure 4b), M-PAT (Figure 4c), M-141 (Figure 4d) and FM3-D (Figure 4f) but not of the gp100 expressing, HLA-A2-negative melanoma G-43 cells (Figure 4e) nor of the HLA-A2-positive, gp100-negative MDA-MB-231 breast carcinoma cells (Figure 4g). The G1 scFv did not stain additional 6 non-melanoma cell lines tested (data not shown).

These data show the ability of the G1 scFv TCR-like antibody to detect specific peptide-MHC complexes *in situ* on cells and potentially in tissue sections after naturally occurring active intracellular processing. This is the first demonstration of *in situ* detection of a tumor-derived T-cell epitope using TCR-like scFv recombinant antibodies.

### EXAMPLE 5

### TCR-LIKE ANTIBODIES CAN INDUCE CELL DEATH IN A PEPTIDE SPECIFIC MHC-COMPLEX - RESTRICTED MANNER

To determine the ability of TCR-like antibodies to induce cell death and eliminate antigen-presenting cells, peptide-loaded APCs were subjected to treatment with the G1 scFv antibody and propidium iodide (PI) or Annexin V staining were employed. PI stains adherent apoptotic cells and Anenxin V detects the membrane exposure to phosphatidyl serine (PS), indicative of early apoptosis events in damaged cells.

### Results

***G1 scFv can induce cell death in melanoma cells loaded with HLA-A2 peptides -*** JY cells were loaded with the gp100-derived epitopes G9-209M and G9-280V as well as with other control HLA-A2-restricted peptides. As is previously mentioned, FACS analysis with anti-HLA-A2 antibody revealed a similar expression pattern of HLA-A2 molecules with G9-209M, G9-280V, and other control peptide-loaded cells (Figures 3a-h). As shown in Figures 5a-d, Annexin V staining revealed that G1 scFv can induce cell death of cells loaded with the specific G9-209 peptide (Figure 5c) but not of cells loaded with a control peptide (Figure 5d). No cytotoxic activity was observed on RMAS-HHD cells that were loaded with the gp100-derived G9-280V epitope or with other control HLA-A2-restricted peptides or cells that were not loaded with peptide (not shown). In the EBV-transformed JY cells, which express normal TAP, the display of the exogenously- supplied peptide is facilitated by peptide exchange (i.e., the peptide from the outside is replacing that peptide that is coming from the inside).

These results demonstrate, for the first time, that TCR-like antibodies can induce cell death in a peptide-specific MHC-restricted manner.

***G1scFv can induce cell death in untreated melanoma cells -*** To further investigate the induced cell death ("the killing effect"), the influence of G1 scFv was tested on human melanoma cell lines established from patients. Melanoma lines which are gp100 and HLA-A2 positive (526, 624, and 501A), gp100 positive but HLA-A2 negative (G43), and gp100 negative and HLA-A2 positive (1938) were subjected to treatment with the G1 antibody and the effect of the antibody on protein synthesis was measured. As shown in Figure 6, the G1 scFv antibody induced cell death, in a dose-dependent manner, of all melanoma cells positive for both the HLA-A2 protein and the tumor specific gp100 protein. On the other hand, as is further shown in Figure 6, protein synthesis of the 1938 or G43 melanoma cell lines which do not express the gp100 antigen or the correct MHC allele, respectively, was not affected by the antibody.

These results demonstrate, for the first time, that a TCR-like antibody, such as the G1, can induce efficient cell death of target cells. These results may pave the way for a new family of antibody-based molecules that can be used as therapeutic agents to eliminate in an antigen (peptide) and MHC-restricted manner specific populations of diseased cells such as tumor cells or virus-infected cells that express disease-specific MHC-peptide complexes.

Since melanoma differentiation antigens-derived T cell epitopes are the most characterized TAAs and many immunotherapy strategies were developed using these targets such as adoptive transfer strategies and vaccination with peptides and dedritic cells (DCs), the present inventors have generated a panel of T-cell receptor like (TCRL) antibodies directed to melanoma differentiation antigens such as gp100, MART1 and tyrosinase and other tumor associated MHC-peptide complexes as well as viral targets (Denkberg, G., et al., 2002, Proc. Natl. Acad. Sci. USA. 99: 9421-9426; Denkberg, G., et 1., 2003, J. Immunol. 171:2197-2207; Lev,A., et al., 2002, Cancer Res. 62: 3184-3194; Cohen, CJ., et al., 2002, Cancer Res. 62: 5835-5844; Cohen, CJ., et al., 2003, J. Immunol 170: 4349-4361). These TCRL antibodies were capable of binding to peptide-pulsed APCs (Figure 1-3) as well as to melanoma cell lines derived from melanoma patients that are HLA-A2 positive (Figures 4a-d). Thus, TCRL antibodies were found capable of binding the authentic MHC-peptide complex as expressed on the cell surface and this reactivity was correlative and dependent on HLA-A2 and antigen expression.

***TCRL-PE fused antibodies exhibit anti-tumor activity -*** TCRL antibodies were genetically fused to a truncated form of Pseudomonas exotoxin (PE38) in which the cell binding domain of the toxin is deleted and the translocation and ADP-ribosylation domains are fused to the TCRL antibody. The effect of such fused TCRL molecules on protein synthesis was tested on cells loaded with various peptides. As shown in Figures 7a-c, a specific protein inhibition activity was achieved when cells were loaded with the specific gp100-derived peptide and thus exhibited the HLA-peptide (e.g., G9-209) complex.

Figures 8a-d depict TCRL internalization into melanoma cells and the specific 2F1 cytotoxicity effect of TCRL (e.g., 2F1 antibody) on HLA-A2+/gp100+ melanoma cells (e.g., 526, 501A, 624.38) but not on the HLA-A2-/gp100+ (G-43) or HLA-A2+/gp100- (1938) melanoma cell-lines. In addition, as is further shown in Figure 8d, such inducement of cell death was achieved in the presence of relatively low TCRL concentrations, with an efficient IC₅₀ of 15-30 ng/ml.

These results strongly suggest the potential use of TCRL antibodies to target drugs or toxins specifically on tumor cells.

***Naked TCRL antibodies exhibit potent inhibition of protein synthesis in an HLA-A2 and antigen specific manner -*** There are strong indications in the literature that antibodies to MHC class I and class II have biological effects on target cells in particular their ability to initiate signal transduction events that will lead to target cell apoptosis (Nagy ZA, et al., 2002; Nagy ZA and Mooney NA., 2003; Longo, D.L., 2002; Vidovic D and Toral JI. 1998; Pedersen AE, et al., 1999; Ruhwald M, et al., 1999; Mori M, et al., 1999; Genestier L, et al., 1997; Genestier L, et al., 1997; Skov S, et al., 1997). These studies were performed with anti-class I and class II antibodies that are specific for MHC but are not allele and peptide specific as the TCRL antibodies of the present invention. To test the ability of the TCRL antibodies of the present invention to exert biological activities toward target cells as naked antibodies, melanoma cell lines derived from patients were incubated with gp100 and MART1-specific TCRL antibodies.

***Naked TCRL antibodies induced apoptosis in melanoma cells in an HLA-peptide specific manner* -** Melanoma cells were subjected to the 2F1, 9H, and G1 naked TCR-like antibody fragments directed to gp100 or MART1 (CLA12) and cell apoptosis was measured using Propidium Iodine (PI) flow cytometry staining. Figures 9a-j depict the results of these experiments. While in cells which were HLA-A2+/antigen+ the fraction of PI positive cells was in the range of 44-58 % (Figures 9e-g), in HLA-A2(+) gp100(-) MART (-) or HLA-A2(-) ge100(+) MART (+) cells, the fraction of PI positive cells was in the range of 7-14 % (Figures 9a-d).

To test the specificity of the naked TCRL antibodies to the HLA-peptide complex, the 624 melanoma cells which are gp100+/HLA-A2+ were treated with the G1 and the control 1A7 (specific to the HLA-A2 in a complex with the mutant G9-209M peptide but not in a complex with the natural G9-209 peptide) TCRL antibodies and the cells were subjected to PI Annexin V flow cytometry analysis. As is shown in Figures 9i-j, while in cells treated with the G1 TCRL the fractions of PI and Annexin V - positive cells were -20 % and 53 %, respectively, in cells treated with the control 1A7 TCRL antibody, the fractions of PI and Annexin V - positive cells where ~7 a% and 12 %, respectively.

***Cloning of TCRL antibodies* -** It is not known from the literature whether the signaling pathways involved through binding of antibodies to class I require dimmerization. The present inventors have hypothesized that the high concentrations of the melanoma-specific TCRL scFv are due to the fact that at high concentrations there is spontaneous dimmer formation. Thus, the TCRL scFv fragment was transformed into whole IgG molecule by cloning the Fd and CL Fab domains into an IgG1 backbone and a whole TCRL IgG molecule was formed in HEK293 cells and purified the TCRL IgG from culture supernatants. Several clones were isolated essentially as described in Thomas Jostocka, et al., Rapid generation of functional human IgG antibodies derived from Fab-on-phage display libraries, Journal of Immunological Methods, Volume 289, Issues 1-2, June 2004, Pages 65-80). ***The cloned TCRL IgG exhibits increased specificity towards the HLA-peptide complex* -** The TCR-like antibody 9H, targeting gp100 HLA-A2/G9-209 peptide, was transformed into a whole IgG molecule and its reactivity on peptide-pulsed JY cells was tested. As shown in Figures 10a-d, the reactivity and specificity of the TCRL IgG molecules to APCs pulsed with the specific gp100-derived melanoma peptide was maintained and even improved due to the increase in its avidity. Similarly, an IgG clone was prepared from the 2F1 scFv, which is directed against the HLA-A2-G9-280 complex. Table 2, hereinbelow, presents a comparison between the activity of naked TCRL IgG molecules and the scFv molecules on melanoma cell lines. As is shown in Table 2, the activity of cell death inducement was maintained in the cloned TCRLs. Moreover, the concentration of TCRL IgG molecule required to achieve a significant level of 50 % cell death was significantly reduced compared to the scFv antibody.

**Table 2**

| ***Induction of cell death in tumor cells by TCR-like antibodies: The influence of antibody avidity monovalent versus bivalent*** | | | |
|---|---|---|---|
| ***Percent specific cell killing (cell death)*** | ***scFv antibody*** | ***IgG Whole antibody*** | ***Fold change*** |
| 20 | 37 µg/ml | 0.5 µg/ml | 74-fold |
| 30 | 75 µg/ml | 2.5 µg/ml | 30-fold |
| 50 | 130 µg/ml | 4 µg/ml | 33-fold |

| | | | |
|---|---|---|---|
| Table 2: Concentration of 2F1 scFv antibody (monovalent) and whole 2F1 IgG (bivalent) required to achieve specific cell death of melanoma cells with a TCR-like antibody targeting the gp100-G9-280 melanoma epitope. | | | |

These results demonstrate the potential use of TCRL antibodies to serve as targeting moieties to melanoma cells as well as considering their potential applications as molecules that may induce specific MHC-restricted, peptide-specific apoptosis in target cells and may be considered as a new entity that may be developed pre clinically as a therapeutic immunotherapeutic modality to melanoma and other tumor types.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

### REFERENCES

### (Additional references are cited in text)

1. Aharoni R., Teitelbaum D., Arnon R., Puri J. 1991. Immunomodulation of experimental allergic encephalomyelitis by antibodies to the antigen-Ia complex. Nature, 351: 147-150.
2. Altman J.D., Moss P.A.H., Goulder P.J.R., Barouch D.H., McHeyzer-Williams M.G., Bell J.I., McMichael A.J., Davis M.M. 1996. Phenotypic analysis of antigen-specific T lymphocytes [published erratum appears in Science 1998 Jun 19;280(5371):1821]. Science, 274: 94-96.
3. Andersen P.S., Stryhn A., Hansen B.E., Fugger L., Engberg J., Buus S. 1996. A recombinant antibody with the antigen-specific, major histocompatibility complex-restricted specificity of T cells. Proc. Natl. Acad. Sci. U. S. A, 93: 1820-1824.
4. Boon T.van der B.P. 1996. Human tumor antigens recognized by T lymphocytes. J. Exp. Med., 183: 725-729.
5. Cloutier S.M., Couty S., Terskikh A., Marguerat L., Crivelli V., Pugnieres M., Mani J.C., Leisinger H.J., Mach J.P., Deperthes D. 2000. Streptabody, a high avidity molecule made by tetramerization of in vivo biotinylated, phage display-selected scFv fragments on streptavidin. Mol. Immunol., 37: 1067-1077.
6. Cohen C.J., Hoffmann N., Farago M., Hoogenboom H.R., Eisenbach L., Reiter Y. 2002. Direct detection and quantitation of a distinct T-cell epitope derived from tumor-specific epithelial cell-associated mucin using human recombinant antibodies endowed with the antigen-specific, major histocompatibility complex-restricted specificity of T cells. Cancer Res., 62: 5835-5844.
7. Cohen C.J., Sarig O., Yamano Y., Tomaru U., Jacobson S., Reiter Y. 2003. Direct Phenotypic Analysis of human MHC Class I Antigen Presentation: Visualization, Quantitation, and In Situ Detection of Human Viral Epitopes Using Peptide-Specific, MHC-restricted Human Recombinant Antibodies. J.Immunol. 170: 4349-4361,2003.
8. Dadaglio G., Nelson C.A., Deck M.B., Petzold S.J., Unanue E.R. 1997. Characterization and quantitation of peptide-MHC complexes produced from hen egg lysozyme using a monoclonal antibody. Immunity., 6: 727-738.
9. Day P.M., Yewdell J.W., Porgador A., Germain R.N., Bennink J.R. 1997. Direct delivery of exogenous MHC class I molecule-binding oligopeptides to the endoplasmic reticulum of viable cells. Proc. Natl. Acad. Sci. U. S. A, 94: 8064-8069.
10. Denkberg G., Cohen C.J., Segal D., Kirkin A.F., Reiter Y. 2000. Recombinant human single-chain MHC-peptide complexes made from E. coli by in vitro refolding: functional single-chain MHC-peptide complexes and tetramers with tumor associated antigens. Eur. J Immunol., 30: 3522-3532.
11. Denkberg G., Cohen C.J., Reiter Y. 2001. Critical role for CD8 in binding of MHC tetramers to TCR: CD8 antibodies block specific binding of human tumor-specific MHC-peptide tetramers to TCR. J Immunol., 167: 270-276.
12. Denkberg G., Cohen C.J., Lev A., Chames P., Hoogenboom H.R., Reiter Y. 2002a. Direct visualization of distinct T cell epitopes derived from a melanoma tumor-associated antigen by using human recombinant antibodies with MHC- restricted T cell receptor-like specificity. Proc. Natl. Acad. Sci. U. S. A, 99: 9421-9426.
13. Denkberg G., Klechevsky E., Reiter Y. 2002b. Modification of a tumor-derived peptide at an HLA-A2 anchor residue can alter the conformation of the MHC-peptide complex: probing with TCR- like recombinant antibodies. J. Immunol., 169: 4399-4407.
14. Krogsgaard M., Wucherpfennig K.W., Canella B., Hansen B.E., Svejgaard A., Pyrdol J., Ditzel H., Raine C., Engberg J., Fugger L. 2000. Visualization of myelin basic protein (MBP) T cell epitopes in multiple sclerosis lesions using a monoclonal antibody specific for the human histocompatibility leukocyte antigen (HLA)-DR2-MBP 85-99 complex. J Exp. Med., 191: 1395-1412.
15. Lev A., Denkberg G., Cohen C.J., Tzukerman M., Skorecki K.L., Chames P., Hoogenboom H.R., Reiter Y. 2002. Isolation and characterization of human recombinant antibodies endowed with the antigen-specific, major histocompatibility complex-restricted specificity of T cells directed toward the widely expressed tumor T- cell epitopes of the telomerase catalytic subunit. Cancer Res., 62: 3184-3194.
16. Polakova K., Plaksin D., Chung D.H., Belyakov I.M., Berzofsky J.A., Margulies D.H. 2000. Antibodies directed against the MHC-I molecule H-2D(d) complexed with an antigenic peptide: similarities to a T cell receptor with the same specificity [In Process Citation]. J Immunol., 165: 5703-5712.
17. Porgador A., Yewdell J.W., Deng Y., Bennink J.R., Germain R.N. 1997. Localization, quantitation, and in situ detection of specific peptide- MHC class I complexes using a monoclonal antibody. Immunity., 6: 715-726.
18. Reiter Y., Di Carlo A., Fugger L., Engberg J., Pastan I. 1997. Peptide-specific killing of antigen-presenting cells by a recombinant antibody-toxin fusion protein targeted to major histocompatibility complex/peptide class I complexes with T cell receptor-like specificity. Proc. Natl. Acad. Sci. U. S. A, 94: 4631-4636.
19. Rognan D., Stryhn A., Fugger L., Lyngbaek S., Engberg J., Andersen P.S., Buus S. 2000. Modeling the interactions of a peptide-major histocompatibility class I ligand with its receptors. I. Recognition by two alpha beta T cell receptors. J. Comput. Aided Mol. Des., 14: 53-69.
20. Stryhn A., Andersen P.S., Pedersen L.O., Svejgaard A., Holm A., Thorpe C.J., Fugger L., Buus S., Engberg J. 1996. Shared fine specificity between T-cell receptors and an antibody recognizing a peptide/major histocompatibility class I complex. Proc. Natl. Acad. Sci. U. S. A, 93: 10338-10342.
21. Wulfing C., Pluckthun A. 1994. Correctly folded T-cell receptor fragments in the periplasm of Escherichia coli. Influence of folding catalysts. J. Mol. Biol., 242: 655-669.
22. Zhong G., Reis e Sousa, Germain R.N. 1997a. Antigen-unspecific B cells and lymphoid dendritic cells both show extensive surface expression of processed antigen-major histocompatibility complex class II complexes after soluble protein exposure in vivo or in vitro. J. Exp. Med., 186: 673-682.
23. Zhong G., Reis e Sousa, Germain R.N. 1997b. Production, specificity, and functionality of monoclonal antibodies to specific peptide-major histocompatibility complex class II complexes formed by processing of exogenous protein. Proc. Natl. Acad. Sci. U. S. A, 94: 13856-13861.
24. Yamano Y, Cohen CJ, Takenouchi N, Yao K, Tomaru U, Li HC, Reiter Y, Jacobson S. Increased Expression of Human T Lymphocyte Virus Type I (HTLV-I) Tax11-19 Peptide-Human Histocompatibility Leukocyte Antigen A*201 Complexes on CD4+ CD25+ T Cells Detected by Peptide-specific, Major Histocompatibility Complex-restricted Antibodies in Patients with HTLV-I-associated Neurologic Disease. J Exp Med. 199: 1367-1377,2004.
25. Zehn, D., Cohen, CJ., Reiter, ., and Walden, P. Extended presentation of specific MHC-peptide complexes by mature dendritic cells compared to other types of antigen-presenting cells. Eur. J. Immunol. 34: 1551-1560, 2004.
26. Nagy ZA, Hubner B, Lohning C, Rauchenberger R, Reiffert S, Thomassen-Wolf E, Zahn S, Leyer S, Schier EM, Zahradnik A, Brunner C, Lobenwein K, Rattel B, Stanglmaier M, Hallek M, Wing M, Anderson S, Dunn M, Kretzschmar T, Tesar M. 2002. Fully human, HLA-DR-specific monoclonal antibodies efficiently induce programmed death of malignant lymphoid cells. Nat Med. 8:801-7.
27. Nagy ZA, Mooney NA. 2003. A novel, alternative pathway of apoptosis triggered through class II major histocompatibility complex molecules. J Mol Med. 81:757-65
28. Longo, D.L., 2002. DR's orders: human antibody kills tumors by direct signaling. Nature Med. 8:781-3.
29. Vidovic D, Toral JI. 1998. Selective apoptosis of neoplastic cells by the HLA-DR-specific monoclonal antibody.Cancer Lett. 128:127-35.
30. Pedersen AE, Bregenholt S, Johansen B, Skov S, Claesson MH. 1999.MHC-I-induced apoptosis in human B-lymphoma cells is dependent on protein tyrosine and serine/threonine kinases. Exp Cell Res. 251:128-34.
31. Ruhwald M, Pedersen AE, Claesson MH. 1999. MHC class I crosstalk with CD2 and CD28 induces specific intracellular signalling and leads to growth retardation and apoptosis via a p56(lck)-dependent mechanism. Exp Clin Immunogenet.;16:199-211
32. Mori M, Terui Y, Ikeda M, Tomizuka H, Uwai M, Kasahara T, Kubota N, Itoh T, Mishima Y, Douzono-Tanaka M, Yamada M, Shimamura S, Kikuchi J, Furukawa Y, Ishizaka Y, Ikeda K, Mano H, Ozawa K, Hatake K. 1999. Beta(2)-microglobulin identified as an apoptosis-inducing factor and its characterization.Blood. 94:2744-53.
33. Genestier L, Paillot R, Bonnefoy-Berard N, Meffre G, Flacher M, Fevre D, Liu YJ, Le Bouteiller P, Waldmann H, Engelhard VH, Banchereau J, Revillard JP. 1997. Fas-independent apoptosis of activated T cells induced by antibodies to the HLA class I alpha1 domain. Blood. 90:3629-39.
34. Genestier L, Meffre G, Garrone P, Pin JJ, Liu YJ, Banchereau J, Revillard JP. 1997. Antibodies to HLA class I alpha1 domain trigger apoptosis of CD40-activated human B lymphocytes. Blood. 90:726-35.
35. Skov S, Klausen P, Claesson MH. 1997. Ligation of major histocompatability complex (MHC) class I molecules on human T cells induces cell death through PI-3 kinase-induced c-Jun NH2-terminal kinase activity: a novel apoptotic pathway distinct from Fas-induced apoptosis.J Cell Biol. 139:1523-31.

## Claims

1. An *in vitro* method of inducing apoptosis in cancer cells, the method comprising contacting the cancer cells with a recombinant, isolated toxin-free single chain antibody capable of specifically binding an MHC class I-peptide complex specifically expressed on the cancer cells, thereby inducing apoptosis in the cancer cells.

2. Use of a recombinant, isolated toxin-free antibody capable of specifically binding an MHC class I-peptide complex, said recombinant, isolated toxin-free antibody being capable of inducing apoptosis of cancer cells specifically expressing said MHC-class I-peptide complex, wherein said recombinant, isolated toxin-free antibody is selected from the group consisting of a Fab fragment and a single chain antibody, for the manufacture of a medicament for the treatment of cancer.

3. A pharmaceutical composition for use in treating cancer comprising as an active ingredient a recombinant, isolated toxin-free antibody capable of specifically binding an MHC class I-peptide complex in cancer cells specifically expressing said MHC class I-peptide complex, wherein said recombinant, isolated toxin-free antibody is selected from the group consisting of a Fab fragment and a single chain antibody, said recombinant, isolated toxin-free antibody being capable of inducing apoptosis in said cancer cells and a pharmaceutically acceptable carrier.

4. The method of claim 1, further comprising detecting apoptosis in said cancer cells following said administering or said expressing.

5. The method of claim 4, wherein said detecting apoptosis is effected by a method selected from the group consisting of propidium iodide FACS analysis, Annexin V FACS analysis, Ethidium homodimer-1 staining, live/dead viability/cytotoxicity assay and Tunnel assay.

6. The method of claim 1, the use of claim 2, or the pharmaceutical composition of claim 3, wherein said single chain antibody is 9H set forth in SEQ ID NO:1, G1 set forth in SEQ ID NO:2 or CLA12 set forth in SEQ ID NO:17.

7. The method of claim 1, 4, 5, the use of claim 2, or the pharmaceutical composition of claim 3, wherein said peptide is derived from a polypeptide selected from the group consisting of MUC1, gp100, HTERT, TAX, MART1.

8. The method of claim 1, 4, 5, the use of claim 2, or the pharmaceutical composition of claim 3, wherein said recombinant antibody comprises the six CDRs set forth by SEQ ID NOs:5-10, 11-16 or 19-24.

9. The method of claim 1, 4, 5, the use of claim 2, or the pharmaceutical composition of claim 3, wherein said cancer is melanoma or breast cancer.

10. The method of claim 1, 4, 5, the use of claim 2, or the pharmaceutical composition of claim 3, wherein said cancer is melanoma and wherein said peptide is derived from tyrosinase.

## Patentansprüche

1. *In-vitro*-Verfahren zum Induzieren von Apoptose in Krebszellen, wobei das Verfahren das Inkontaktbringen der Krebszellen mit einem rekombinanten, isolierten toxinfreien Einzelketten-Antikörper umfasst, der in der Lage ist, einen MHC-Klasse-I-Peptidkomplex spezifisch zu binden, der auf den Krebszellen spezifisch exprimiert wird, wodurch in den Krebszellen eine Apoptose induziert wird.

2. Verwendung eines rekombinanten, isolierten toxinfreien Antikörpers, der in der Lage ist, einen MHC-Klasse-I-Peptidkomplex spezifisch zu binden, wobei der rekombinante, isolierte toxinfreie Antikörper in der Lage ist, eine Apoptose von Krebszellen zu induzieren, die den MHC-Klasse-I-Peptidkomplex spezifisch exprimieren, wobei der rekombinante, isolierte toxinfreie Antikörper aus der Gruppe ausgewählt ist, bestehend aus einem Fab-Fragment und einem Einzelketten-Antikörper, zur Herstellung eines Medikaments zur Behandlung von Krebs.

3. Pharmazeutische Zusammensetzung zur Verwendung bei der Behandlung von Krebs, die als Wirkstoff einen rekombinanten, isolierten toxinfreien Antikörper, der in der Lage ist, einen MHC-Klasse-I-Peptidkomplex in Krebszellen spezifisch zu binden, die den MHC-Klasse-I-Peptidkomplex spezifisch exprimieren, wobei der rekombinante, isolierte toxinfreie Antikörper aus der Gruppe ausgewählt ist, bestehend aus einem Fab-Fragment und einem Einzelketten-Antikörper, wobei der rekombinante, isolierte toxinfreie Antikörper in der Lage ist, eine Apoptose in den Krebszellen zu induzieren, und einen pharmazeutisch annehmbaren Träger umfasst.

4. Verfahren nach Anspruch 1, das des Weiteren das Nachweisen von Apoptose in den Krebszellen nach Verabreichung oder Expression umfasst.

5. Verfahren nach Anspruch 4, wobei das Nachweisen der Apoptose durch ein Verfahren erfolgt, das aus der Gruppe ausgewählt ist, bestehend aus Propidiumiodid-FACS-Analyse, Annexin-V-FACS-Analyse, Ethidiumhomodimer-1-Färbung, Live/Dead-Viability/Cytotoxicity-Assay und Tunnel-Assay.

6. Verfahren nach Anspruch 1, Verwendung nach Anspruch 2 oder pharmazeutische Zusammensetzung nach Anspruch 3, wobei der Einzelketten-Antikörper 9H, wie in SEQ ID NO:1 angeführt, G1, wie in SEQ ID NO:2 angeführt, oder CLA12, wie in SEQ ID NO:17 angeführt, ist.

7. Verfahren nach Anspruch 1, 4, 5, Verwendung nach Anspruch 2 oder pharmazeutische Zusammensetzung nach Anspruch 3, wobei das Peptid von einem Polypeptid abgeleitet ist, das aus der Gruppe ausgewählt ist, bestehend aus MUC1, gp100, HTERT, TAX, MART1.

8. Verfahren nach Anspruch 1, 4, 5, Verwendung nach Anspruch 2 oder pharmazeutische Zusammensetzung nach Anspruch 3, wobei der rekombinante Antikörper die sechs CDRs, wie in SEQ ID NOs:5-10, 11-16 oder 19-24 angeführt, umfasst.

9. Verfahren nach Anspruch 1, 4, 5, Verwendung nach Anspruch 2 oder pharmazeutische Zusammensetzung nach Anspruch 3, wobei der Krebs ein Melanom oder Brustkrebs ist.

10. Verfahren nach Anspruch 1, 4, 5, Verwendung nach Anspruch 2 oder pharmazeutische Zusammensetzung nach Anspruch 3, wobei der Krebs ein Melanom ist, und wobei das Peptid von Tyrosinase abgeleitet ist.

## Revendications

1. Méthode *in vitro* d'induction de l'apoptose dans des cellules cancéreuses, la méthode consistant à mettre en contact les cellules cancéreuses avec un anticorps à chaîne unique, exempt de toxine, isolé, recombinant capable de se lier spécifiquement à un complexe du peptide I de classe MHC spécifiquement exprimé sur les cellules cancéreuses, induisant ainsi l'apoptose dans les cellules cancéreuses.

2. Utilisation d'un anticorps exempt de toxine, isolé, recombinant capable de se lier spécifiquement à un complexe du peptide I de classe MHC, ledit anticorps exempt de toxine, isolé, recombinant étant capable d'induire l'apoptose de cellules cancéreuses, en particulier en exprimant ledit complexe du peptide I de classe MHC, dans laquelle ledit anticorps exempt de toxine, isolé, recombinant est choisi dans le groupe constitué par un fragment Fab et un anticorps à chaîne unique, pour la fabrication d'un médicament destiné au traitement d'un cancer.

3. Composition pharmaceutique pour une utilisation dans le traitement d'un cancer comprenant en tant que principe actif un anticorps exempt de toxine, isolé, recombinant capable de se lier spécifiquement à un complexe du peptide I de classe MHC dans des cellules cancéreuses, en particulier en exprimant ledit complexe du peptide I de classe MHC, dans laquelle ledit anticorps exempt de toxine, isolé, recombinant est choisi dans le groupe constitué par un fragment Fab et un anticorps à chaîne unique, ledit anticorps exempt de toxine, isolé, recombinant étant capable d'induire l'apoptose dans lesdites cellules cancéreuses et un véhicule pharmaceutiquement acceptable.

4. Méthode selon la revendication 1, consistant en outre à détecter l'apoptose dans lesdites cellules cancéreuses après ladite administration ou ladite expression.

5. Méthode selon la revendication 4, dans laquelle ladite détection de l'apoptose est réalisée par une méthode choisie dans le groupe constitué par l'analyse FACS à l'iodure de propidium, l'analyse FACS à l'annexine V, la coloration à l'homodimère 1 d'éthidium, un dosage de viabilité/cytotoxicité des cellules mortes/vivantes et un dosage Tunnel.

6. Méthode selon la revendication 1, utilisation selon la revendication 2 ou composition pharmaceutique selon la revendication 3, dans laquelle ledit anticorps à chaîne unique est 9H décrit dans la SEQ ID N° 1 ; G1 décrit dans la SEQ ID N° 2 ou CLA12 décrit dans la SEQ ID N° 17.

7. Méthode selon la revendication 1, 4; 5, utilisation selon la revendication 2 ou composition pharmaceutique selon la revendication 3, dans laquelle ledit peptide est dérivé d'un polypeptide choisi dans le groupe constitué par MUC1, gp100, HTERT, TAX, MART1.

8. Méthode selon la revendication 1, 4, 5, utilisation selon la revendication 2 ou composition pharmaceutique selon la revendication 3, dans laquelle ledit anticorps recombinant comprend les six séquences CDR décrites dans les SEQ ID N° 5 à 10, 11 à 16 ou 19 à 24.

9. Méthode selon la revendication 1, 4, 5, utilisation selon la revendication 2 ou composition pharmaceutique selon la revendication 3, dans laquelle ledit cancer est un mélanome ou un cancer du sein.

10. Méthode selon la revendication 1, 4, 5, utilisation selon la revendication 2 ou composition pharmaceutique selon la revendication 3, dans laquelle ledit cancer est un mélanome et dans laquelle ledit peptide est dérivé de la tyrosinase.


### SEQUENCE LISTING

<110> Reiter, Yoram Kalchevsky, Eynav Denkberg, Galit
<120> ANTIBODIES FOR SELECTIVE APOPTOSIS OF CELLS
<130> 29525
<160> 24
<170> PatentIn version 3.3
<210> 1
   <211> 248
   <212> PRT
   <213> Artificial sequence
<220>
   <223> Single chain Fv 9H
<220>
   <221> misc_feature
   <222> (1)..(120)
   <223> VH fragment
<220>
   <221> misc_feature
   <222> (31)..(36)
   <223> CDR
<220>
   <221> misc_feature
   <222> (50)..(66)
   <223> CDR
<220>
   <221> misc_feature
   <222> (99)..(109)
   <223> CDR
<220>
   <221> misc_feature
   <222> (121)..(136)
   <223> Single chain Fv linker
<220>
   <221> misc_feature
   <222> (137)..(248)
   <223> VL fragment
<220>
   <221> misc_feature
   <222> (160)..(172)
   <223> CDR
<220>
   <221> misc_feature
   <222> (188)..(198)
   <223> CDR
<220>
   <221> misc_feature
   <222> (227)..(237)
   <223> CDR
<400> 1
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Gln Tyr Tyr
               20                  25                  30
   Ala Met Ser Trp Val Arg Gln Ala Pro Gly Lys Gly Leu Glu Trp Val
           35                  40                  45
   Ser Ser Ile Asn Trp Asn Ser Gly Thr Leu Asp Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Tyr Ser Asn Tyr Gly Leu Tyr Ala Met Asp Val Trp Gly Gln
               100                 105                 110
   Gly Thr Leu Val Thr Val Ser Ser Ser Gly Gly Gly Gly Ser Gly Gly
           115                 120                 125
   Gly Gly Ser Gly Gly Gly Gly Ser Ser Asp Ile Val Leu Thr Gln Pro
       130                 135                 140
   Pro Ser Ala Ser Gly Thr Pro Gly Gln Arg Val Thr Ile Ser Cys Ser
   145                 150                 155                 160
   Gly Ser Ser Ser Asn Ile Gly Ser Asn Pro Val Thr Trp Tyr Gln Gln
                   165                 170                 175
   Leu Pro Gly Thr Ala Pro Lys Pro Leu Ile Tyr Gly Asp Ser Tyr Arg
               180                 185                 190
   Pro Ser Gly Val Pro Asp Arg Phe Ser Gly Ser Lys Ser Gly Thr Ser
           195                 200                 205
   Ala Ser Leu Ala Ile Ser Gly Leu Arg Ser Glu Asp Glu Ala Asp Tyr
       210                 215                 220
   Tyr Cys Ala Thr Trp Asp Asp Asp Val Asn Gly Val Ile Phe Gly Gly
   225                 230                 235                 240
   Gly Thr Gln Leu Thr Val Leu Arg
                   245
<210> 2
   <211> 237
   <212> PRT
   <213> Artificial sequence
<220>
   <223> Single chain Fv G1
<220>
   <221> misc_feature
   <222> (1)..(118)
   <223> VH fragment
<220>
   <221> misc_feature
   <222> (31)..(35)
   <223> CDR
<220>
   <221> misc_feature
   <222> (50)..(66)
   <223> CDR
<220>
   <221> misc_feature
   <222> (99)..(108)
   <223> CDR
<220>
   <221> misc_feature
   <222> (119)..(134)
   <223> Single chain Fv linker
<220>
   <221> misc_feature
   <222> (135)..(237)
   <223> VL fragment
<220>
   <221> misc_feature
   <222> (158)..(167)
   <223> CDR
<220>
   <221> misc_feature
   <222> (183)..(189)
   <223> CDR
<220>
   <221> misc_feature
   <222> (222)..(230)
   <223> CDR
<400> 2
   Gln Val Lys Leu Gln Glu Ser Gly Gly Gly Leu Val Lys Pro Gly Gly
   1               5                   10                  15
   Ser Leu Lys Leu Ser Cys Ala Ala Ser Gly Phe Thr Phe Ser Ser Tyr
               20                  25                  30
   Gly Met Ser Trp Val Arg Gln Thr Pro Asp Lys Arg Leu Glu Trp Val
           35                  40                  45
   Ala Thr Ile Ser Ser Gly Gly Ser Tyr Thr Tyr Tyr Pro Asp Ser Val
       50                  55                  60
   Lys Gly Arg Phe Thr Ile Ser Arg Asp Asn Ala Lys Asn Thr Leu Tyr
   65                  70                  75                  80
   Leu Gln Met Ser Ser Leu Lys Ser Glu Asp Thr Ala Met Tyr Tyr Cys
                   85                  90                  95
   Ala Arg Gly Asn Trp Glu Gly Trp Tyr Phe Asp Val Trp Gly Gln Gly
               100                 105                 110
   Thr Thr Val Thr Val Ser Ser Gly Gly Gly Gly Ser Gly Gly Gly Gly
           115                 120                 125
   Ser Gly Gly Gly Gly Ser Asn Ile Glu Leu Thr Gln Ser Pro Ala Ile
       130                 135                 140
   Met Ser Ala Ser Pro Gly Glu Arg Val Thr Met Thr Cys Ser Ala Ser
   145                 150                 155                 160
   Ser Ser Ile Arg Tyr Ile Tyr Trp Tyr Gln Gln Lys Pro Gly Ser Ser
                   165                 170                 175
   Pro Arg Leu Leu Ile Tyr Asp Thr Ser Asn Val Ala Pro Gly Val Pro
               180                 185                 190
   Phe Arg Phe Ser Gly Ser Gly Ser Gly Thr Ser Tyr Ser Leu Thr Ile
           195                 200                 205
   Asn Arg Met Glu Ala Glu Asp Ala Ala Thr Tyr Tyr Cys Gln Glu Trp
       210                 215                 220
   Ser Gly Tyr Pro Tyr Thr Phe Gly Gly Gly Thr Lys Leu
   225                 230                 235
<210> 3
   <211> 744
   <212> DNA
   <213> Artificial sequence
<220>
   <223> Single chain Fv 9H coding sequence
<220>
   <221> misc_feature
   <222> (1) .. (360)
   <223> VH fragment coding sequence
<220>
   <221> misc_feature
   <222> (91)..(105)
   <223> CDR
<220>
   <221> misc_feature
   <222> (148)..(198)
   <223> CDR
<220>
   <221> misc_feature
   <222> (295)..(327)
   <223> CDR
<220>
   <221> misc_feature
   <222> (361)..(408)
   <223> Single chaine Fv linker coding sequence
<220>
   <221> misc_feature
   <222> (409)..(744)
   <223> VL fragment coding sequence
<220>
   <221> misc_feature
   <222> (478)..(516)
   <223> CDR
<220>
   <221> misc_feature
   <222> (562).. (594)
   <223> CDR
<220>
   <221> misc_feature
   <222> (679)..(711)
   <223> CDR
<400> 3
   gaggtgcagc tgttggagtc tgggggaggc ttggtacagc ctggggggtc cctgagactc      60
   tcctgtgcag cctctggatt cacctttcag tactatgcca tgagttgggt ccgccaggct     120
   ccagggaagg ggctggagtg ggtctcaagt attaattgga atagtggtac cttagactat     180
   gcggactctg tgaagggccg attcaccatc tccagagaca attccaagaa cacgctgtat     240
   ctgcaaatga acagcctgag agccgaggac acggccgtgt attactgtgc aagatacagt     300
   aactacggac tctacgcaat ggacgtctgg ggccagggca ccctggtcac ggtctcttca     360
   tcaggtggag gcggttcagg cggaggtggc tctggcggtg gcggatcgtc cgatatcgtg     420
   ctgactcagc caccctcagc gtctgggacc cccgggcaga gggtcaccat ctcttgctct     480
   ggaagcagct ccaacatcgg aagtaatcct gttacctggt accagcagct tccaggaacg     540
   gcccccaaac ccctcattta tggtgacagt tatcggccct caggggtccc tgaccggttc     600
   tctggctcca agtctggcac ctcagcctcc ctggccatca gtgggctgcg gtccgaggat     660
   gaggctgatt attactgtgc cacatgggat gacgatgtga atggtgtaat attcggcgga     720
   gggacccagc tcaccgtcct ccgt                                            744
<210> 4
   <211> 711
   <212> DNA
   <213> Artificial sequence
<220>
   <223> Single chain Fv G1 coding sequence
<220>
   <221> misc_feature
   <222> (1)..(354)
   <223> VH fragment coding sequence
<220>
   <221> misc_feature
   <222> (91)..(105)
   <223> CDR
<220>
   <221> misc_feature
   <222> (148)..(198)
   <223> CDR
<220>
   <221> misc_feature
   <222> (295)..(324)
   <223> CDR
<220>
   <221> misc_feature
   <222> (355)..(378)
   <223> Single chain Fv linker coding sequence
<220>
   <221> misc_feature
   <222> (379)..(711)
   <223> VL fragment coding sequence
<220>
   <221> misc_feature
   <222> (472)..(501)
   <223> CDR
<220>
   <221> misc_feature
   <222> (547)..(567)
   <223> CDR
<220>
   <221> misc_feature
   <222> (664)..(690)
   <223> CDR
<400> 4
   caggtgaaac tgcaggagtc tgggggaggc ttagtgaagc ctggagggtc cctgaaactc     60
   tcctgtgcag cctctggatt cactttcagt agctatggca tgtcttgggt tcgccagact    120
   ccagacaaga ggctggagtg ggtcgcaacc attagtagtg gtggtagtta cacctactat    180
   ccagacagtg tgaaggggcg attcaccatc tccagagaca atgccaagaa caccctgtac    240
   ctgcaaatga gcagtctgaa gtctgaggac acagccatgt attactgtgc aagaggtaac    300
   tgggaaggat ggtacttcga tgtctggggc caagggacca cggtcaccgt ctcctcaggt    360
   ggaggcggtt caggcggagg tggctctggc ggtggcggat cgaacatcga gctcactcag    420
   tctccagcaa tcatgtctgc atctccaggg gagagggtca ccatgacctg cagtgccagc    480
   tcaagtatac gttacatata ttggtaccaa cagaagcctg gatcctcccc cagactcctg    540
   atttatgaca catccaacgt ggctcctgga gtcccttttc gcttcagtgg cagtgggtct    600
   gggacctctt attctctcac aatcaaccga atggaggctg aggatgctgc cacttattac    660
   tgccaggagt ggagtggtta tccgtacacg ttcggagggg ggacaaagtt g             711
<210> 5
   <211> 5
   <212> PRT
   <213> Artificial sequence
<220>
   <223> scFv 9H CDR
<400> 5
   Tyr Tyr Ala Met Ser
   1 5
<210> 6
   <211> 17
   <212> PRT
   <213> Artificial sequence
<220>
   <223> scFv 9H CDR
<400> 6
   Ser Ile Asn Trp Asn Ser Gly Thr Leu Asp Tyr Ala Asp Ser Val Lys
   1 5 10 15
   Gly
<210> 7
   <211> 11
   <212> PRT
   <213> Artificial sequence
<220>
   <223> scFv 9H CDR
<400> 7
   Tyr Ser Asn Tyr Gly Leu Tyr Ala Met Asp Val
   1 5 10
<210> 8
   <211> 13
   <212> PRT
   <213> Artificial sequence
<220>
   <223> scFv 9H CDR
<400> 8
   Ser Gly Ser Ser Ser Asn Ile Gly Ser Asn Pro Val Thr
   1 5 10
<210> 9
   <211> 11
   <212> PRT
   <213> Artificial sequence
<220>
   <223> scFv 9H CDR
<400> 9
   Gly Asp Ser Tyr Arg Pro Ser Gly Val Pro Asp
   1 5 10
<210> 10
   <211> 11
   <212> PRT
   <213> Artificial sequence
<220>
   <223> scFv 9H CDR
<400> 10
   Ala Thr Trp Asp Asp Asp Val Asn Gly Val Ile
   1 5 10
<210> 11
   <211> 5
   <212> PRT
   <213> Artificial sequence
<220>
   <223> scFv G1 CDR
<400> 11
   Ser Tyr Gly Met Ser
   1               5
<210> 12
   <211> 17
   <212> PRT
   <213> Artificial sequence
<220>
   <223> scFv G1 CDR
<400> 12
   Thr Ile Ser Ser Gly Gly Ser Tyr Thr Tyr Tyr Pro Asp Ser Val Lys
   1               5                   10                  15
   Gly
<210> 13
   <211> 10
   <212> PRT
   <213> Artificial sequence
<220>
   <223> scFv G1 CDR
<400> 13
   Gly Asn Trp Glu Gly Trp Tyr Phe Asp Val
   1               5                   10
<210> 14
   <211> 10
   <212> PRT
   <213> Artificial sequence
<220>
   <223> scFv G1 CDR
<400> 14
   Ser Ala Ser Ser Ser Ile Arg Tyr Ile Tyr
   1               5                   10
<210> 15
   <211> 7
   <212> PRT
   <213> Artificial sequence
<220>
   <223> scFv G1 CDR
<400> 15
   Asp Thr Ser Asn Val Ala Pro
   1               5
<210> 16
   <211> 9
   <212> PRT
   <213> Artificial sequence
<220>
   <223> scFv G1 CDR
<400> 16
   Gln Glu Trp Ser Gly Tyr Pro Tyr Thr
   1               5
<210> 17
   <211> 238
   <212> PRT
   <213> Artificial sequence
<220>
   <223> Single chain Fv CLA12
<220>
   <221> misc_feature
   <222> (1)..(111)
   <223> VH fragment
<220>
   <221> misc_feature
   <222> (30) .. (37)
   <223> CDR
<220>
   <221> misc_feature
   <222> (52)..(67)
   <223> CDR
<220>
   <221> misc_feature
   <222> (100)..(111)
   <223> CDR
<220>
   <221> misc_feature
   <222> (112)..(127)
   <223> Single chain Fv linker
<220>
   <221> misc_feature
   <222> (128)..(238)
   <223> VL fragment
<220>
   <221> misc_feature
   <222> (150)..(162)
   <223> CDR
<220>
   <221> misc_feature
   <222> (178)..(184)
   <223> CDR
<220>
   <221> misc_feature
   <222> (217).. (236)
   <223> CDR
<400> 17
   Gln Leu Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Ile Ser Ser Ser
               20                  25                  30
   Ser Tyr Tyr Trp Gly Trp Ile Arg Gln Pro Pro Gly Lys Gly Leu Glu
           35                  40                  45
   Trp Ile Gly Ser Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg His Val Gly His Glu Leu Asp Tyr Trp Gly Gln Gly Ser
               100                 105                 110
   Gly Gly Gly Gly Ser Gly Gly Gly Gly Ser Gly Gly Gly Gly Ser Gln
           115                 120                 125
   Ser Val Val Thr Gln Pro Pro Ser Val Ser Ala Ala Pro Gly Arg Lys
       130                 135                 140
   Val Thr Ile Ser Cys Ser Gly Ser Ser Ser Asn Ile Gly Ser Asn Tyr
   145                 150                 155                 160
   Val Ser Trp Tyr Gln Gln Val Pro Gly Thr Ala Pro Lys Leu Leu Ile
                   165                 170                 175
   Tyr Glu Asp Asp Lys Arg Pro Ser Gly Ile Pro Asp Arg Phe Ser Gly
               180                 185                 190
   Ser Lys Ser Gly Thr Ser Ala Thr Leu Gly Ile Thr Gly Leu Gln Thr
           195                 200                 205
   Gly Asp Glu Ala Asp Tyr Phe Cys Ala Thr Trp Asp Arg Thr Val Asn
       210                 215                 220
   Val Val Arg Phe Gly Gly Gly Thr Arg Leu Thr Val His Ser
   225                 230                 235
<210> 18
   <211> 714
   <212> DNA
   <213> Artificial sequence
<220>
   <223> Single chain Fv CLA12 coding sequence
<220>
   <221> misc_feature
   <222> (1)..(333)
   <223> VH fragment coding sequence
<220>
   <221> misc_feature
   <222> (334)..(378)
   <223> Single chain FV linker coding sequence
<220>
   <221> misc_feature
   <222> (379)..(714)
   <223> VL fragment coding sequence
<400> 18
   cagctgcagc tgcaggagtc gggcccagga ctggtgaagc cttcggagac cctgtccctc     60
   acctgcactg tctctggtgg ctccatcagc agtagtagtt actactgggg ctggatccgc    120
   cagcccccag ggaaggggct ggagtggatt gggagtatct attatagtgg gagcacctac    180
   tacaacccgt ccctcaagag tcgagtcacc atatccgtag acacgtccaa gaaccagttc    240
   tccctgaagc tgagctctgt gaccgccgca gacacggctg tgtattactg tgcgagacat    300
   gtgggccacg aacttgacta ctggggccag ggatcaggtg gaggcggttc aggcggaggt    360
   ggctctggcg gtggcggatc gcagtctgtc gtgacgcagc cgccctcagt gtctgcggcc    420
   ccaggacgga aggtcaccat ctcctgctct ggaagcagct ccaacattgg gagtaattat    480
   gtgtcctggt accagcaagt cccagggaca gcccccaaac tcctcatcta tgaagatgat    540
   aagcgaccct cagggattcc tgaccgattc tctggctcca agtctgggac gtccgccacc    600
   ctgggcatca ccggactaca gactggggac gaggccgatt atttctgcgc cacgtgggat    660
   aggactgtca atgttgtacg tttcggcgga gggaccaggc tgaccgtcca cagt          714
<210> 19
   <211> 7
   <212> PRT
   <213> Artificial sequence
<220>
   <223> scFv CLA12 CDR
<400> 19
   Ser Ser Ser Tyr Tyr Trp Gly
   1               5
<210> 20
   <211> 16
   <212> PRT
   <213> Artificial sequence
<220>
   <223> scFv CLA12 CDR
<400> 20
   Ser Ile Tyr Tyr Ser Gly Ser Thr Tyr Tyr Asn Pro Ser Leu Lys Ser
   1               5                   10                  15
<210> 21
   <211> 12
   <212> PRT
   <213> Artificial sequence
<220>
   <223> scFv CLA12 CDR
<400> 21
   His Val Gly His Glu Leu Asp Tyr Trp Gly Gln Gly
   1               5                   10
<210> 22
   <211> 13
   <212> PRT
   <213> Artificial sequence
<220>
   <223> scFv CL12 CDR
<400> 22
   Ser Gly Ser Ser Ser Asn Ile Gly Ser Asn Tyr Val Ser
   1               5                   10
<210> 23
   <211> 7
   <212> PRT
   <213> Artificial sequence
<220>
   <223> scFv CLA12 CDR
<400> 23
   Glu Asp Asp Lys Arg Pro Ser
   1               5
<210> 24
   <211> 20
   <212> PRT
   <213> Artificial sequence
<220>
   <223> scFv CLA12 CDR
<400> 24
   Ala Thr Trp Asp Arg Thr Val Asn Val Val Arg Phe Gly Gly Gly Thr
   1               5                   10                  15
   Arg Leu Thr Val
               20